# EUROPEAN PATENT APPLICATION

(11) **EP 4 717 191 A1**
(43) Date of publication of application: **01.04.2026**
(21) Application number: 24848201.0
(22) Date of filing: 28.07.2024
(51) Int. Cl.: A61B 17/072, A61B 17/068, A61B 17/115

(54) **SURGICAL INSTRUMENT AND END EFFECTOR ASSEMBLY THEREOF**

(30) Priority: 28.07.2023 CN 202310946119; 28.07.2023 CN 202310947483; 28.07.2023 CN 202310942800
(71) Applicant: Reach Surgical, Inc., Tianjin 300462 (CN)
(72) Inventor: LI, Shuaishuai, Tianjin 300462 (CN)
(74) Representative: Dantz, Jan Henning
(86) International application number: PCT/CN2024/108020
(87) International publication number: WO 2025/026246

(57) **Abstract**

The present invention relates to a surgical instrument (100) and an end effector assembly (30) thereof. The end effector assembly includes: a proximal body portion (30a) comprising a articulating member (36) and a firing member (35); a distal execution portion (30b) comprising a staple cartridge assembly (31) and a staple anvil assembly (32), the distal execution portion (30b) being pivotally connected to the proximal body portion (30a) through an articulation joint assembly (38), wherein the articulation joint assembly (38) includes a proximal connecting member (381a) fixedly connected to a distal end of the proximal body portion (30a); a distal connecting member (382a) fixedly connected to a proximal end of the distal execution portion (30b), wherein the distal connecting member (382a) is engaged with the proximal connecting member (381a), and the distal connecting member (382a) is provided with at least one articulation driving portion (383a), and the articulating member (36) operably drives the articulation driving portion (383a) to move, so as to cause the distal execution portion (30b) to articulate relative to the proximal body portion (30a); and a retainer (384) pivotally connected to the proximal connecting member (381a) and the distal connecting member (382a) separately, the retainer (384) having a retaining channel (43) for receiving the firing member (35).

## Description

### Technical field

The present invention relates to the field of surgical instruments, and in particular to a surgical instrument for clamping, cutting and stapling, and an end effector assembly thereof.

### Technical background

Surgical stapling instruments are commonly used operating instruments in laparoscopic surgery, and are suitable for stapling and cutting tissue. The instrument usually includes a handle assembly, an elongated body assembly and an end effector assembly. When used during surgery, a part of the elongated body assembly and the end effector assembly enter a patient's body through an access port established by a trocar. A surgeon can operate the handle assembly to articulate the end effector assembly to a certain angle relative to the elongated body assembly, so as to adapt to various positions for tissue cutting and stapling. In some specific surgical scenarios, it is often required that the end effector assembly can provide a larger articulation angle to achieve a smaller incision and fewer staple placement positions, thereby reducing the probability of anastomotic leakage.

To increase the articulation angle of the end effector assembly, dual or even multiple joints are used in prior art to achieve an end effector assembly with large-angle articulation. Although a larger articulation angle can be provided by using a progressive articulating manner with multiple pivot shafts, the length of the joint portions is also increased, enlarging the articulating radius of the end effector assembly and making it difficult to adapt to confined surgical scenarios such as a pelvic cavity. In the existing designs, an end effector that articulates on a single pivot is also used. A firing member composed of multiple metal sheets undergoes a large articulation at the single pivot. For example, when the end effector is articulated to 90° angle, the firing member also needs to be articulated to 90° at the single pivot. When a surgical operation is carried out in this state, the firing member is prone to popping out of the joint or pivot or becoming stacked at the joint or pivot in the case that there is no limiting mechanism or the limiting mechanism has an unreasonable structure.

### Summary

To this end, the present invention proposes a surgical instrument with a small articulating radius that can adapt to a confined space and with a firing member that can be moved reliably.

In view of the above technical problem, the present invention provides the following technical solutions:

An end effector assembly of a surgical instrument, comprising: a proximal body portion defining a longitudinal axis and comprising an articulating member and a firing member; a distal execution portion comprising a staple cartridge assembly and a staple anvil assembly, opposing surfaces of the staple cartridge assembly and the staple anvil assembly forming a clamping face for clamping tissue, and the distal execution portion being pivotally connected to the proximal body portion through an articulation joint assembly, wherein the articulation joint assembly comprises a proximal connecting member fixedly connected to a distal end of the proximal body portion; and a distal connecting member fixedly connected to a proximal end of the distal execution portion, wherein the distal connecting member is engaged with the proximal connecting member, and the distal connecting member is provided with at least one articulation driving portion, and the articulating member operably drives the articulation driving portion to move, so as to cause the distal execution portion to articulate relative to the proximal body portion; and a retainer pivotally connected to the proximal connecting member and the distal connecting member separately, the retainer having a retaining channel for receiving the firing member.

In some implementations of the present invention, a distal end of the proximal connecting member has a toothed structure, and a proximal end of the distal connecting member has a toothed structure that meshes with the proximal connecting member; and a position in which the distal connecting member meshes with the proximal connecting member forms a pivot point of the distal execution portion, and when the distal execution portion extends along the direction of the longitudinal axis, the articulation driving portion is arranged so as not to overlap with the longitudinal axis.

In some implementations of the present invention, the retainer is separately provided with a proximal pivot shaft and a distal pivot shaft; the proximal pivot shaft of the retainer is pivotally engaged with a proximal pivot hole of the proximal connecting member, and the distal pivot shaft of the retainer is pivotally engaged with a distal pivot hole of the distal connecting member; and a line connecting axes of the proximal pivot shaft and the distal pivot shaft is located in the retaining channel.

In some implementations of the present invention, the articulation joint assembly further comprises a first connecting piece, the first connecting piece is provided with two connecting holes, and a proximal pivot shaft and a distal pivot shaft of the retainer respectively pass through a proximal pivot hole of the proximal connecting member and a distal pivot hole of the distal connecting member and are riveted to the connecting holes of the connecting member.

In some implementations of the present invention, the distal connecting member is provided with a first articulation driving portion and a second articulation driving portion, and the articulating member is separately connected to the first articulation driving portion and the second articulation driving portion through a transmission assembly.

In some implementations of the present invention, the transmission assembly comprises: a first rack and a first articulation transmission member fixedly connected to the first rack, wherein a proximal end of the first rack is connected to the articulating member, and the first articulation transmission member is connected to one of the articulation driving portions of the distal connecting member; a second rack and a second articulation transmission member fixedly connected to the second rack, wherein the second rack is arranged opposite to the first rack with a spacing therebetween, and the second articulation transmission member is connected to the other one of the articulation driving portions of the distal connecting member; and at least one gear located between the first rack and the second rack and separately meshing with the first rack and the second rack.

In some implementations of the present invention, the first articulation driving portion and the second articulation driving portion are configured as pivot pins fixedly connected to the distal connecting member, and the first articulation transmission member or the second articulation transmission member is riveted or sleeved on the respective pivot pin.

In some implementations of the present invention, the first articulation driving portion of the distal connecting member is configured as a pivot pin with an axial direction perpendicular to the clamping face, and a distal end of the first articulation transmission member is provided with a connecting sleeve, and the first articulation transmission member is sleeved on the first articulation driving portion through the connecting sleeve.

In some implementations of the present invention, the second articulation driving portion of the distal connecting member is configured as a stud with an axial direction parallel to the clamping face, a distal end of the second articulation transmission member is provided with a rivet hole, and the second articulation driving portion is connected to the second articulation transmission member through the rivet hole.

In some implementations of the present invention, the retaining channel is a unidirectional curved channel, in which a first support wall and a second support wall defining the retaining channel are curved in the same direction, and when the firing member is articulated to a maximum angle position, the first support wall is adapted to support an outer side wall of the firing member, and the second support wall is adapted to support an inner side wall of the firing member.

In some implementations of the present invention, an arc length of the first support wall is less than an arc length of the second support wall.

In some implementations of the present invention, a part of the retainer where the first support wall is located is a first support region, and a part of the retainer where the second support wall is located is a second support region, wherein a proximal end face of the first support region is located distal to a proximal end face of the second support region, and a distal end face of the first support region is located proximal to a distal end face of the second support region.

In some implementations of the present invention, when the distal execution portion extends along the direction of the longitudinal axis, a distal edge of the first support wall is substantially aligned with an inner wall of a firing channel of the distal execution portion; and a proximal end of the first support wall is substantially aligned with an inner wall of a channel supporting relative sliding of the firing member in the proximal body portion.

In some implementations of the present invention, when the distal execution portion extends along the direction of the longitudinal axis, a distal end of the first support wall and a proximal end of a firing channel of the distal connecting member are in a clearance fit, and a proximal end of the first support wall and a distal end of a firing channel of the proximal connecting member are in a clearance fit.

In some implementations of the present invention, the proximal connecting member is provided with a first limiting protrusion on a side surface facing the retainer, the first limiting protrusion being opposite to a proximal end face of the first support region with a first gap therebetween; and the distal connecting member is provided with a second limiting protrusion on a side surface facing the retainer, the second limiting protrusion being opposite to a distal end face of the first support region with a second gap therebetween.

In some implementations of the present invention, the first limiting protrusion and the second limiting protrusion each have a first side surface arranged opposite to the first support region and a second side surface arranged opposite to the firing member, and an included angle between the first side surface and the second side surface is between 80° and 100°.

In some implementations of the present invention, when the distal execution portion extends along the direction of the longitudinal axis, the second side surfaces of the first limiting protrusion and the second limiting protrusion are parallel to the longitudinal axis.

In some implementations of the present invention, when the distal execution portion extends along the direction of the longitudinal axis, the first side surface of the first limiting protrusion is arranged parallel to the proximal end face of the first support region of the retainer, and the first side surface of the second limiting protrusion is parallel to the distal end face of the first support region of the retainer.

In some implementations of the present invention, the retaining channel is a bidirectional curved channel, in which a first support wall and a second support wall defining the retaining channel are each arc-shaped with a direction of curvature that is convex outward, and when the distal execution portion is articulated relative to the proximal body portion, the first support wall or the second support wall supports an outer arc articulated surface of the firing member.

In some implementations of the present invention, a part of the retainer where the first support wall is located is a first support region, an outer side wall of the first support region is a first outer arc wall, the first outer arc wall has a direction of curvature same as that of the first support wall and an arc length greater than that of the first support wall, and the first outer arc wall and the first support wall are transitioned at both ends through first transition walls, respectively; and a part of the retainer where the second support wall is located is a second support region, an outer side wall of the second support region is a second outer arc wall, the second outer arc wall has a direction of curvature same as that of the second support wall and an arc length greater than that of the second support wall, and the second outer arc wall and the second support wall are transitioned at both ends through second transition walls, respectively.

In some implementations of the present invention, when the distal execution portion is articulated to a maximum articulation angle relative to the proximal body portion, the first transition wall or the second transition wall on a proximal side of the retainer limits a proximal inner arc surface position of the firing member, and the first transition wall or the second transition wall on a distal side of the retainer limits a distal inner arc surface position of the firing member.

In some implementations of the present invention, a portion of the firing member that engages with the first transition wall or the second transition wall on the proximal side is a curved transition region on a proximal side of the firing member, and a portion of the firing member that engages with the first transition wall or the second transition wall on the distal side is a curved transition region on a distal side of the firing member.

In some implementations of the present invention, when the distal execution portion is articulated to a maximum articulation angle relative to the proximal body portion, a proximal end of the first transition wall or the second transition wall on the proximal side substantially abuts against an inner wall of a firing channel of the proximal connecting member so as to limit articulation of the firing member, and a distal end of the first transition wall or the second transition wall on the distal side substantially abuts against an inner wall of a firing channel of the distal connecting member so as to limit articulation of the firing member.

In some implementations of the present invention, when the distal execution portion extends along longitudinal axis, distal edges of the first support wall and the second support wall and an inner wall of a firing channel of the distal execution portion are substantially aligned in a straight line; and proximal ends of the first support wall and the second support wall and an inner wall of a channel supporting relative sliding of the firing member in the proximal body portion are substantially aligned in a straight line.

In some implementations of the present invention, when the end effector assembly is in a ready-to-load position, the distal execution portion forms, with respect to the longitudinal axis, an included angle that is not 0°.

In some implementations of the present invention, the proximal body portion further comprises a locking member rotatably sleeved on a proximal side thereof, the locking member comprises a first locking portion, and when the end effector assembly is in the ready-to-load position, the first locking portion engages with the articulating member, so as to lock the articulating member.

In some implementations of the present invention, the locking member comprises a second locking portion, and when the end effector assembly is in the ready-to-load position, the second locking portion engages with the firing member, so as to lock the firing member.

In some implementations of the present invention, the first locking portion of the locking member is engaged in a locked position with the articulating member, and the second locking portion of the locking member is engaged in a locked position with the firing member, respectively, by means of rotational insertion.

In some implementations of the present invention, the locking member is molded/formed as a semi-annular sleeve with a notch, the first locking portion is a first protrusion extending circumferentially along a notched side wall of the locking member, and the second locking portion is a second protrusion extending inwardly along an inner wall of the locking member.

In some implementations of the present invention, the proximal body portion comprises a support main body, the locking member is sleeved on a proximal end of the support main body, and the first locking portion and the second locking portion are each located at a distal end of the locking member.

In some implementations of the present invention, the locking member further comprises an unlocking driving portion, and when the end effector assembly is in a loading position, the unlocking driving portion is subjected to a circumferential force to drive the locking member to rotate and disengage from the articulating member and the firing member.

The present invention also provides a surgical instrument, comprising a handle assembly, an elongated body assembly, and an end effector assembly selectively engaged with the elongated body assembly, which are sequentially connected from a proximal end to a distal end, wherein the end effector assembly is implemented as described herein.

The technical effects of the technical solution(s) of the present invention will be described in detail in the following specific embodiments.

### Brief description of the drawings

Preferred embodiments in the present invention will be described in detail below with reference to the accompanying drawings, which will help to understand the objectives and advantages of the present invention. In the accompanying drawings:
Fig. 1 is a schematic structural view of a specific embodiment of a surgical instrument of the present invention;
Fig. 2 is a schematic structural view of a specific embodiment of the end effector assembly of the surgical instrument of the present invention;
Fig. 3 is an exploded view of the specific embodiment of the end effector assembly of the surgical instrument of the present invention;
Fig. 4 is an exploded view of a portion of the structure of a specific embodiment of the end effector assembly of the present invention;
Fig. 5 is an exploded view of a portion of the structure of a specific embodiment of the end effector assembly of the present invention;
Fig. 6 is a schematic structural view of a specific embodiment of a retainer in the surgical instrument of the present invention;
Fig. 7 is a schematic view of a specific embodiment of a first distal connecting member in the surgical instrument of the present invention;
Fig. 8 is a schematic view of a distal execution portion in the surgical instrument of the present invention when extending along the direction of a longitudinal axis;
Fig. 9 is an enlarged view of a portion of the structure of Fig. 8;
Fig. 10 is a schematic view of a portion of an articulation joint assembly of the distal execution portion of the present invention when extending along the direction of the longitudinal axis;
Fig. 11 is a schematic view of the distal execution portion in the surgical instrument of the present invention when articulated to a maximum articulation angle;
Fig. 12 is an enlarged view of a portion of the structure of Fig. 11;
Fig. 13 is a schematic view of a portion of an articulation joint assembly of the distal execution portion of the present invention at a maximum articulation angle;
Fig. 14 is a schematic structural view of the end effector assembly of the present invention in an unloaded state;
Fig. 15 is an enlarged view of a portion of the structure of Fig. 14;
Fig. 16 is a schematic view of a portion of the articulation joint assembly of the end effector assembly of the present invention in an unloaded state;
Fig. 17 is a schematic view of the portion of the articulation joint assembly of the end effector assembly of the present invention in a articulated state;
Fig. 18 is a schematic view of another specific embodiment of a lower proximal connecting member of the present invention;
Fig. 19 is a schematic view of another specific embodiment of a lower distal connecting member of the present invention;
Fig. 20 is an enlarged view of a portion of the structure of a distal execution portion in another specific embodiment of the surgical instrument of the present invention when extending along the direction of a longitudinal axis;
Fig. 21 is a schematic view of a portion of an articulation joint assembly of a distal execution portion in another specific embodiment of the present invention when extending along the direction of the longitudinal axis;
Fig. 22 is a schematic view of a specific embodiment of the end effector assembly of the surgical instrument of the present invention;
Fig. 23 is an enlarged view of a portion of Fig. 4;
Fig. 24 is a schematic view of a specific embodiment of a first support main body in the end effector assembly of the present invention;
Fig. 25 is a schematic view of a portion of the structure of a firing member in the end effector assembly of the present invention;
Fig. 26 is a schematic structural view of the firing member in the end effector assembly of the present invention;
Fig. 27 is an enlarged view of a portion of the structure of Fig. 26;
Fig. 28 is a schematic structural view of a firing connecting member in the end effector assembly of the present invention;
Fig. 29 is a schematic view of the connection between an end effector assembly and an elongated body assembly of the surgical instrument of the present invention;
Fig. 30 is a schematic structural view of a locking member in the end effector assembly of the present invention;
Fig. 31 is a schematic structural view of the locking member in the end effector assembly of the present invention when located in a locked position;
Fig. 32 is a schematic structural view of the locking member in the end effector assembly of the present invention when located in an unlocked position;
Fig. 33 is another structural form of a first articulation transmission member and a second articulation transmission member in the end effector assembly of the present invention;
Fig. 34 is a schematic view of a distal execution portion in another embodiment of the end effector assembly of the present invention when extending along the direction of a longitudinal axis;
Fig. 35 is an enlarged view of a portion of the structure of Fig. 34;
Fig. 36 is an exploded view of a portion of the structure of a second specific embodiment of the end effector assembly of the present invention;
Fig. 37 is an exploded view of a portion of the structure of a second specific embodiment of the end effector assembly of the present invention;
Fig. 38 is a schematic structural view of a second specific embodiment of the retainer in the surgical instrument of the present invention;
Fig. 39 is a top view of the second specific embodiment of the retainer in the surgical instrument of the present invention;
Fig. 40 is a schematic view of a specific embodiment of the first distal connecting member in the surgical instrument of the present invention;
Fig. 41 is a schematic view of a distal execution portion in the surgical instrument of the present invention when extending along the direction of a longitudinal axis;
Fig. 42 is an enlarged view of a portion of the structure of Fig. 41;
Fig. 43 is a schematic view of a portion of an articulation joint assembly of the distal execution portion of the present invention when extending along the direction of the longitudinal axis;
Fig. 44 is a schematic view of the distal execution portion in the surgical instrument of the present invention when articulated to a maximum articulation angle;
Fig. 45 is an enlarged view of a portion of the structure of Fig. 44;
Fig. 46 is a schematic view of the portion of the articulation joint assembly of the distal execution portion of the present invention at a maximum articulation angle; and
Fig. 47 is another structural form of the first articulation transmission member and the second articulation transmission member in the end effector assembly of the present invention.

### Detailed description of the embodiments

The technical solutions of the present invention will be clearly and completely described below with reference to the accompanying drawings. Obviously, the described embodiments are a part, but not all, of the embodiments of the present invention. All other embodiments obtained by an ordinary person skilled in the art on the basis of the embodiments of the present invention without creative efforts are within the scope of protection of the present invention.

In the description of the embodiments of the present invention, it should be note that the orientation or positional relationships indicated by the terms such as "center", "upper", "lower", "left", "right", "vertical", "horizontal", "inner", "outer", and so on, are based on the orientation or positional relationship shown in the drawings, and are only for the purpose of the convenience of describing the present invention and simplifying the description, instead of indicating or implying that the device or element referred to must have a specific orientation or be constructed and operated in a specific orientation. Therefore, these terms cannot be construed as a limitation of the present invention. In addition, the terms "first", "second" and "third" are only used for descriptive purposes, and cannot be understood as the indication or implication of relative importance.

In the description of the present invention, it should also be noted that unless otherwise clearly specified and limited, the terms such as "mounted", "coupled" and "connected" should be understood in a broad sense. For example, "connected" may be a fixed connection, or may be a detachable connection or an integral connection; and it may be a direct connection, or may be an indirect connection through an intermediate medium, or may be an internal communication of two elements. For those of ordinary skill in the art, the specific meaning of the above-mentioned terms in the present invention can be understood according to specific situations.

In addition, the technical features involved in the different implementations of the present invention to be described below can be combined with each other as long as there is no conflict between them. In the following description, identical or similar elements are represented by the same reference signs.

In various embodiments of the present invention, a "distal end/side" refers to an end of a surgical instrument that is away from an operator during operation, and a "proximal end/side" refers to an end/side of the surgical instrument that is close to the operator during operation.

The following is a specific embodiment of the surgical instrument. Generally speaking, the embodiment of the surgical instrument described herein is an endoscopic surgical cutting and stapling instrument. However, it should be pointed out that the surgical instrument may also be a non-endoscopic surgical cutting and stapling instrument, such as an open surgical instrument used in open surgery.

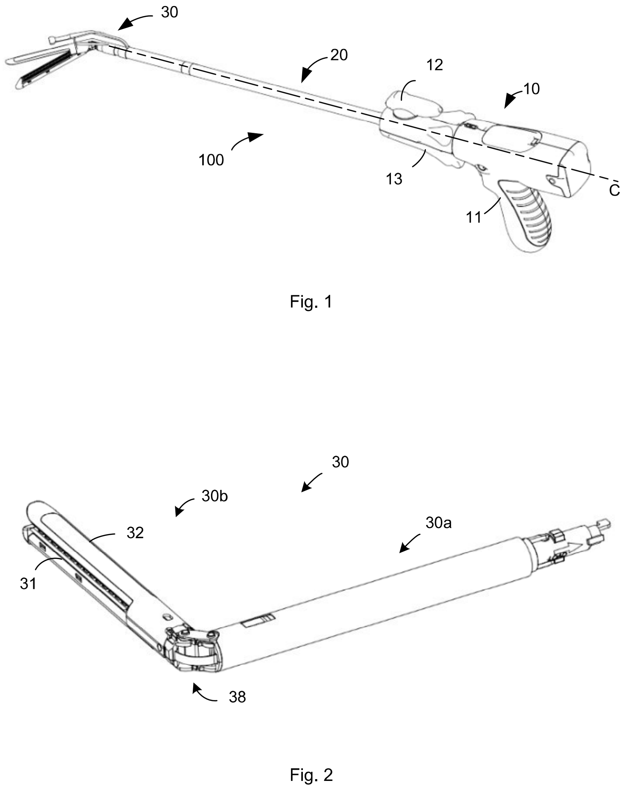

The surgical instrument 100 shown in Fig. 1 includes a handle assembly 10, an elongated body assembly 20, and an end effector assembly 30. The handle assembly 10 is adapted to allow an operator to manipulate the surgical instrument 100. The handle assembly 10 may control the movement of the end effector assembly 30 through the elongated body assembly 20, to perform a surgical operation, such as clamping/closing, suturing/stapling or cutting tissue.

The handle assembly 10 includes a handle housing 11 that can be held by a user in a conventional manner. In a specific implementation, the surgical instrument 100 is manipulated by a trigger to close and fire the end effector assembly 30. In a specific implementation, the surgical instrument 100 may also be manipulated by means of a push knob, a button or the like provided on the handle assembly to close and fire the end effector assembly 30, so that the end effector assembly 30 performs cutting and suturing operations. In other alternative implementations, the surgical instrument 100 may also be manipulated by means of a trigger, a push knob, a button or the like provided on the handle assembly 10 to open jaws of the end effector assembly 30 so as to release the tissue. The handle housing 11 is generally T-shaped as a whole, and includes a main body portion extending along a longitudinal axis C and a holding portion extending approximately in a direction perpendicular to the longitudinal axis C or inclined at an angle relative to the longitudinal axis C. A mounting space for a driving mechanism is formed inside the main body portion and the holding portion.

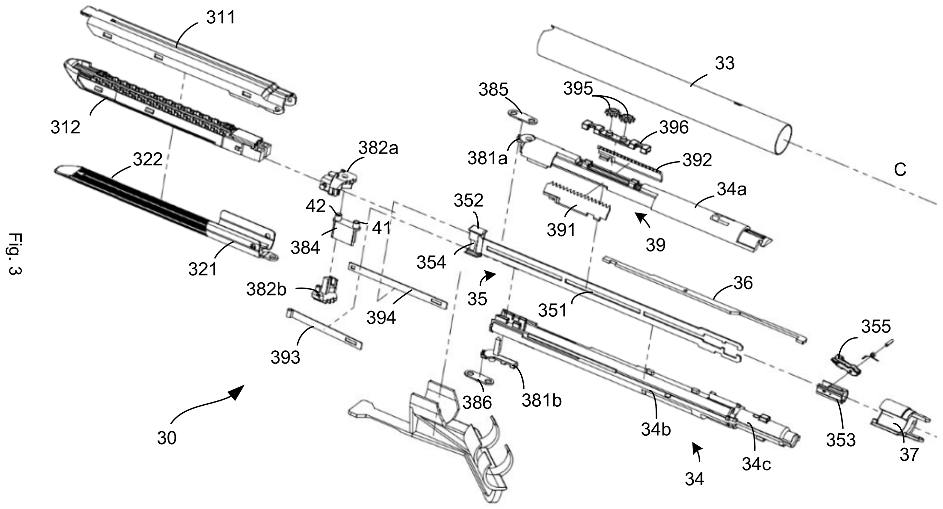

As shown in Fig. 1, the elongated body assembly 20 includes a tubular housing 21 defining a longitudinal axis C. A set of transmission rods (not shown in the figure) is provided within the tubular housing 21. The proximal end of the set of the transmission rods is connected to an output end of a driving mechanism within the handle assembly 10. As shown in Fig. 3, the distal end of the set of the transmission rods is connected to a firing member 35 and an articulating member 36 of the end effector assembly 30 for transmitting a driving force of the driving mechanism to the end effector assembly 30. Specifically, the set of the transmission rods includes a firing rod and an articulation driving rod. The firing rod is configured to transmit the driving force for driving of firing to the firing member 35 of the end effector assembly 30, to implement the firing operation of the end effector assembly 30. The articulation driving rod is configured to transmit the driving force of the articulation driving assembly to the articulating member 36 of the end effector assembly 30, to articulate the end effector assembly 30.

As shown in Fig. 1, the surgical instrument 100 described in an embodiment of the present invention further includes a rotation knob 13. The rotation knob 13 is mounted on the distal side of the handle assembly 10 and is arranged at the proximal end of the elongated body assembly 20. When the rotation knob 13 is operated to rotate about the longitudinal axis C of the surgical instrument 100, it can drive the elongated body assembly 20 and the end effector assembly 30 to rotate together.

The end effector assembly 30 is configured to operate tissue to perform a specific surgical operation, such as clamping, suturing/stapling or cutting of tissue. To realize the articulation of the end effector assembly 30 at a set angle relative to the longitudinal axis C of the elongated body assembly 20, as shown in Fig. 2, the end effector assembly 30 includes a proximal body portion 30a and a distal execution portion 30b. The proximal body portion 30a and the distal execution portion 30b are pivotally connected through an articulation joint assembly 38. Correspondingly, the surgical instrument 100 further includes an articulation driving assembly and an articulation transmission assembly that actuate the articulation joint assembly 38 to articulate. As shown in Fig. 3, the articulation transmission assembly includes an articulating member 36 operably connected to the articulation driving assembly. The articulation driving assembly includes an articulation knob 12 mounted on the rotation knob 13 and an articulation driving rod (not shown) arranged within the elongated body assembly 20. The articulation driving rod is connected to the articulating member 36. Rotating the articulation knob 12 by the operator may drive the articulation driving rod to move. Specifically, when the articulation knob 12 is manipulated to rotate clockwise from an initial position, the articulation driving rod drives the articulating member 36 to move toward the distal end; and when the articulation knob 12 is manipulated to rotate counterclockwise from the initial position, the articulation driving rod drives the articulating member 36 to move toward the proximal end; and vice versa.

A specific embodiment of the end effector assembly 30 of the surgical instrument 100 of the present invention will be described in detail below with reference to Fig. 2. The end effector assembly 30 is removably mounted on the distal end of the elongated body assembly 20 of the surgical instrument 100. The proximal body portion 30a of the end effector assembly 30 may be inserted into the elongated body assembly 20 in a plug-in manner, and be rotated relative to the housing of the elongated body assembly 20 to lock the end effector assembly 30 thereto. The distal execution portion 30b includes a staple cartridge assembly 31 and a staple anvil assembly 32. The staple cartridge assembly 31 and staple anvil assembly 32 are movable relative to each other to close the jaws and thereby clamp tissue jaws. Opposing surfaces of the staple cartridge assembly 31 and staple anvil assembly 32 form a clamping face for clamping tissue. In a specific implementation, the staple anvil assembly 32 is operable to pivot toward the staple cartridge assembly 31 so as to close the jaws of the end effector assembly 30 to clamp tissue; and the staple anvil assembly 32 is then operable to pivot away from the staple cartridge assembly 31 so as to open the jaws of the end effector assembly 30 to release tissue. In an alternative implementation, the staple cartridge assembly 31 of the end effector assembly 30 is operable to pivot toward the staple anvil assembly 32 until the jaws of the end effector assembly 30 are closed to clamp tissue; and the staple cartridge assembly 31 is operable to pivot away from the staple cartridge assembly 31 until the jaws of the end effector assembly 30 are opened to release tissue.

Specifically, as shown in Figs. 2 and 3, the proximal body portion 30a of the end effector assembly 30 is detachably connected to the distal end of the elongated body assembly 20, and the proximal body portion 30a includes an elongated outer tube 33, a support main body 34 arranged in the outer tube 33, a firing member 35 slidably arranged in the support main body 34, and an articulating member 36, wherein the outer tube 33 defines a longitudinal axis C extending in the same direction as the tubular housing 21 of the elongated body assembly 20; the support main body 34 includes a first half support main body 34a and a second half support main body 34b, the proximal end of the second half support main body 34b includes an engagement portion 34c for being connected to the elongated body assembly 20, and an engagement lug is arranged on the engagement portion 34c for releasably engaging with the elongated body assembly 20 in a snap-fit connection manner. Specifically, a connecting piece 361 is provided at the proximal end of the articulating member 36 of the end effector assembly 30, and is engaged with a hook portion at the distal end of the articulation driving rod located inside the elongated body assembly 20. A firing connecting member 353 is arranged at the proximal end of the firing member 35 in the end effector assembly 30, and is engaged with the distal end of a firing rod of the elongated body assembly 20, thereby realizing the rotational engagement and locking between the end effector assembly 30 and the elongated body assembly 20.

A channel for slidably receiving the firing member 35 is provided between the first half support main body 34a and the second half support main body 34b. The firing member 35 includes an elongated firing beam 351. The firing beam 351 may be composed of a single sheet of material or from multiple stacked sheets. A working portion 352 of the firing member 35 is formed into an I-beam structure, and a part of a region thereof abuts against a staple-pushing wedge and may slide integrally toward the distal end of the staple cartridge assembly 31 to perform a corresponding surgical operation. For example, when the firing member 35 is actuated to move from the proximal end to the distal end, the part of the region of the working portion 352 of the firing member 35 moves toward the distal end together with the staple-pushing wedge. The staple-pushing wedge acts on a staple driver to push a staple out of the staple cartridge assembly 31 to implement a tissue stapling operation. Meanwhile, a cutting knife 354 on the working portion 352 cuts tissue. A firing connection portion 353 is arranged at the proximal end of the firing beam 351. The firing connection portion 353 is molded/formed as a sleeve structure with an opening. An aperture is provided at the proximal end of the firing connection portion 353, and is configured to receive the distal end of the firing rod 22 when the proximal end of the end effector assembly 30 is engaged with the elongated body assembly 20.

As shown in Fig. 3, in the distal execution portion 30b of the end effector assembly 30, the staple cartridge assembly 31 includes a staple cartridge 312, a staple cartridge base 311, and a staple-pushing wedge arranged in a cavity between the staple cartridge 312 and the staple cartridge base 311. The proximal end of the firing member 35 is connected to the firing rod within the elongated body assembly 20, and the distal end of the firing member 35 abuts against the staple-pushing wedg and may slide/move integrally along the longitudinal axis to perform a corresponding surgical operation. The staple cartridge assembly 31 further includes staple-pushing members and staples within the staple cartridge 312. When the firing member 35 is actuated to move from the proximal end to the distal end, the staple-pushing wedge is pushed to move, and the staple-pushing wedge acts on the staple-pushing members to push staples out of the staple cartridge 312, realizing an operation of stapling tissue. The staple anvil assembly 32 includes a staple anvil housing 321 and a staple-supporting seat 322 located inside the staple anvil housing 321. The staple-supporting seat 322 is cooperatively engaged with the staple cartridge 312 to realize an operation of forming the staples. The surface of the staple-supporting seat 322 is provided with a plurality of pockets. The pockets are in one-to-one correspondence with the positions of staple holes on the staple cartridge 312. When tissue is to be stapled, the respective staples in the staple holes abut against the respective pockets.

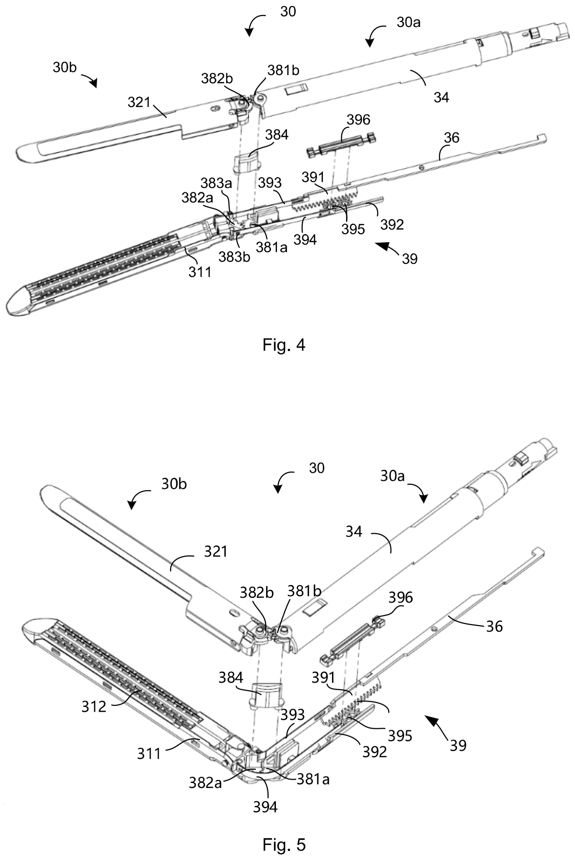

With reference to Figs. 3 to 5, the articulation joint assembly 38 includes a proximal connecting member 381a fixedly connected to the distal end of the proximal body portion 30a, and a distal connecting member 382a fixedly connected to the distal execution portion 30b. Specifically, the proximal connecting member 381a is fixedly connected to the support main body 34 through a matching structure of a positioning protrusion and a positioning slot, or the proximal connecting member 381a and the support main body 34 are integrally formed by a process such as welding or injection molding. The distal connecting member 382a is fixedly connected to the distal execution portion 30b by a positioning pin. As shown in Fig. 4, the distal end of the proximal connecting member 381a and the proximal end of the distal connecting member 382a have toothed structures that mesh with each other, and the meshing position of the two forms a pivot point A of the distal execution portion 30b. The distal connecting member 382a is provided with at least one articulation driving portion 383a connected to the articulating member 36. When the articulating member 36 slides relative to the support main body 34, it may directly or indirectly act on the articulation driving portion 383a of the distal connecting member 382a, so that the distal connecting member 382a can swing around the meshing position between the distal connecting member 382a and the distal connecting member 382a under the action of the meshing teeth, thereby realizing the articulation of the distal execution portion 30b relative to the proximal body portion 30a. When the distal execution portion 30b extends along the direction of the longitudinal axis C (as shown in Fig. 4), the articulation driving portion 383a is spaced apart from the longitudinal axis C and located at the distal end of the pivot point A, that is, the articulation driving portion 383a does not coincide with the longitudinal axis C.

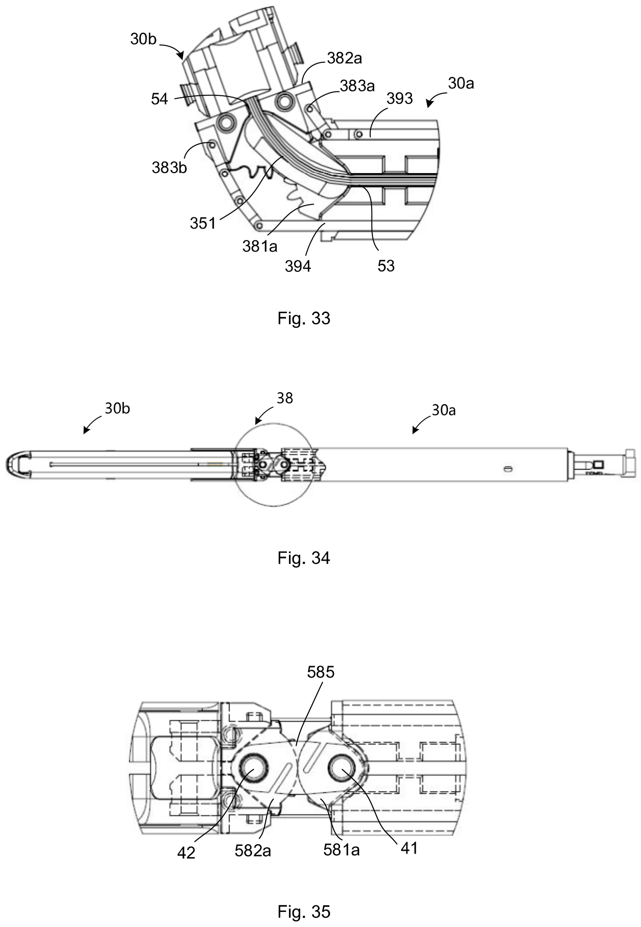

The proximal connecting member 381a and the distal connecting member 382a may also be connected in other engagement manners to allow the distal connecting member 382a to articulate or swing around the proximal connecting member 381a. At the same time, the two can provide relatively stable support, reducing the shaking caused by articulating or swinging during the firing process of the surgical instrument, and avoiding pulling and damaging tissue. Figs. 34 and 35 show a proximal connecting member 581a and a distal connecting member 582a according to another embodiment. The proximal connecting member 581a and the distal connecting member 582a are each provided with an engagement portion in regions opposite to each other. The engagement portion is configured as a friction wheel with a large friction force. The proximal connecting member 581a and the distal connecting member 582a are connected through a connecting piece 585 having a groove/slit. The connecting piece 585 is configured in the form of a spring sheet with a certain elastic deformation. Under the action of an external force, the groove/slit can be expanded or contracted to realize its elastic deformation. The connecting piece 585 is provided with two connecting holes, which are connected to the proximal connecting member 581a and the distal connecting member 582a through shaft pins, respectively. In an initial state, the distance between the two connecting holes of the connecting piece 585 is slightly smaller than the axial spacing between the proximal connecting member 581a and the distal connecting member 582a. After the connecting piece 585 is separately connected to the proximal connecting member 581a and the distal connecting member 582a, the connecting piece 585 is stretched and deformed, and the contraction tension force acts on the proximal connecting member 581a and the distal connecting member 582a, so that the proximal connecting member 581a and the distal connecting member 582a are in close contact, so as to increase the relative friction force and ensure the positioning of the articulation position of the distal connecting member 582a.

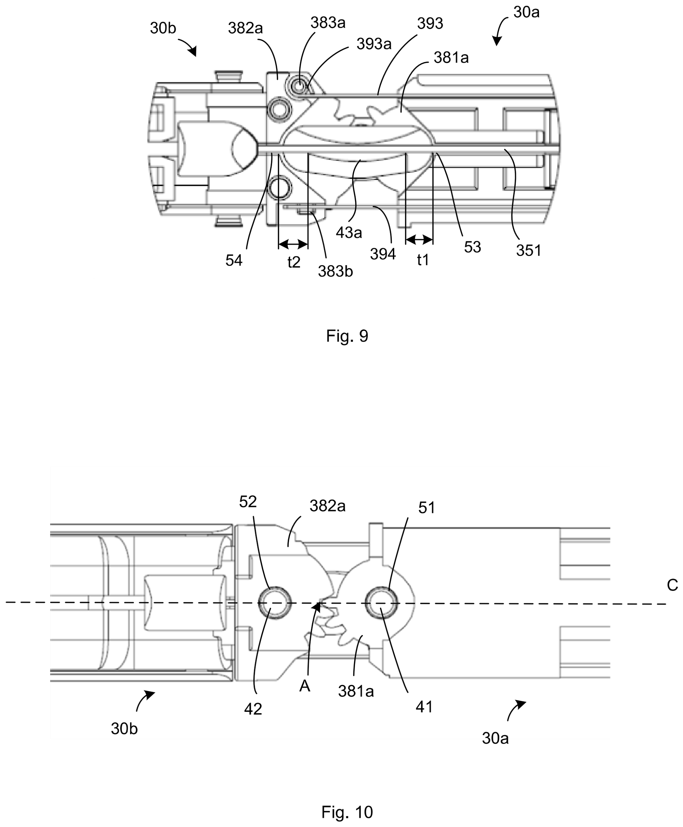

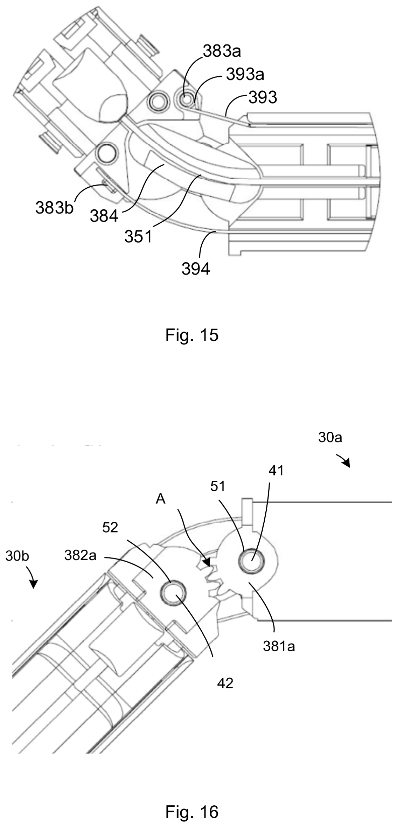

To ensure the stability of the firing member 35 moving toward the distal end after the distal execution portion 30b is articulated relative to the proximal body portion 30a, and to avoid the problem that the articulation force of the firing member 35 (i.e., a biasing force that causes the firing member 35 to articulate and deform) is too large, causing a part of the sheet-like firing beam 351 to pop out or stack, as shown in Figs. 3 to 6, the articulation joint assembly 38 further includes a retainer 384 for accommodating the articulation position of the firing member 35. The retainer 384 is connected to the proximal connecting member 381a and the distal connecting member 382a engaged with each other through two pivot shafts. As shown specifically in Figs. 10, 13, and 16, the retainer 384 is connected to a proximal pivot hole 51 of the proximal connecting member 381a through a proximal pivot shaft 41 and to a distal pivot hole 52 of the distal connecting member 382a through a distal pivot shaft 42, enabling the retainer, with the distal connecting member 382a, to pivot and swing about the proximal pivot shaft 41 relative to the proximal connecting member 381a. The articulation joint assembly 38 further includes a first connecting piece 385. End portions of the pivot shaft 41 and the pivot shaft 42 of the retainer 384 are riveted to the first connecting piece 385. As shown specifically in Figs. 2 and 3, the first connecting piece 385 is provided with two connecting holes, and the end portions of the proximal pivot shaft 41 and the distal pivot shaft 42 of the retainer 384 are riveted to the connecting holes of the first connecting piece 385. The retainer 384 has a retaining channel 43, which is suitable for accommodating at least a part of the firing beam 351 of the firing member 35, so that the firing beam passes through the retaining channel 43 and may move along an extension direction thereof.

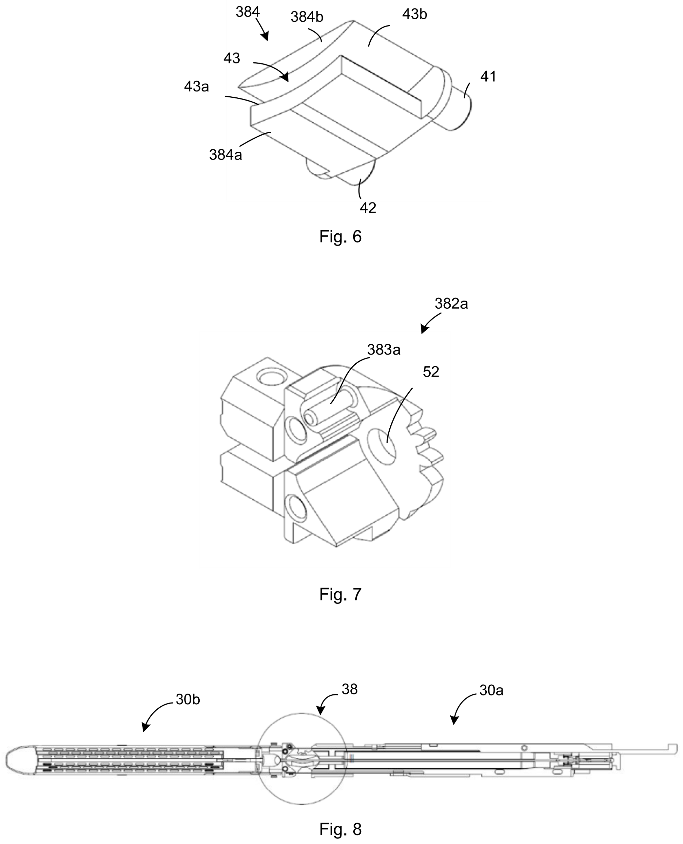

Figs. 4 to 21 show a specific implementation of the end effector assembly 30 of the present invention. In this implementation, the end effector assembly 30 includes a retainer 384, and its retaining channel 43 is configured as an arc structure as shown in Fig. 6. The retaining channel 43 is defined by a first support wall 43a and a second support wall 43b located on both sides of the firing beam 351, and the first support wall 43a and the second support wall 43b are arc-shaped with the same direction of curvature. The first support wall 43a is adapted to engage with a maximum articulation outer arc surface of the firing beam 351, and the second support wall 43b is adapted to engage with a maximum articulation inner arc surface of the firing beam 351.

As shown in Fig. 9, the proximal connecting member 381a includes a firing channel 53 with a proximal end opposite to the firing channel of the proximal body portion 30a and a distal end opposite to the retaining channel 43 of the retainer 384. Correspondingly, the distal connecting member 382a also has a firing channel 54 with a distal end opposite to the firing channel of the distal body portion 30a and a proximal end opposite to the retaining channel 43 of the retainer 384. When the distal execution portion 30b extends along the direction of the longitudinal axis C, the firing channel 53 of the proximal connecting member 381a and the firing channel 54 of the distal connecting member 382a extend in the same direction and are positioned opposite to each other, which is suitable for the firing beam 351 to extend in a straight line. Since both the first support wall 43a and the second support wall 43b of the retaining channel 43 articulate toward the same side, the firing beam 351 of the firing member 35 is allowed to unilaterally articulate to the greatest possible angle. When the firing beam 351 is at its maximum unilateral articulation angle, inner and outer arc surfaces thereof are each provided with better support and position limiting, enabling the distal execution portion 30b to realize a greater unilateral articulation angle. It can be understood that although both the first support wall 43a and the second support wall 43b of the retaining channel 43 articulate toward the same side to provide support and position limiting for the firing beam 351 when the distal execution portion 30b is at its maximum unilateral articulation position, the retaining channel 43 has a certain width, which still allows the distal execution portion 30b to articulate in the opposite direction to a certain angle, thereby enabling the end effector assembly 30 to realize an overall asymmetric articulation angle.

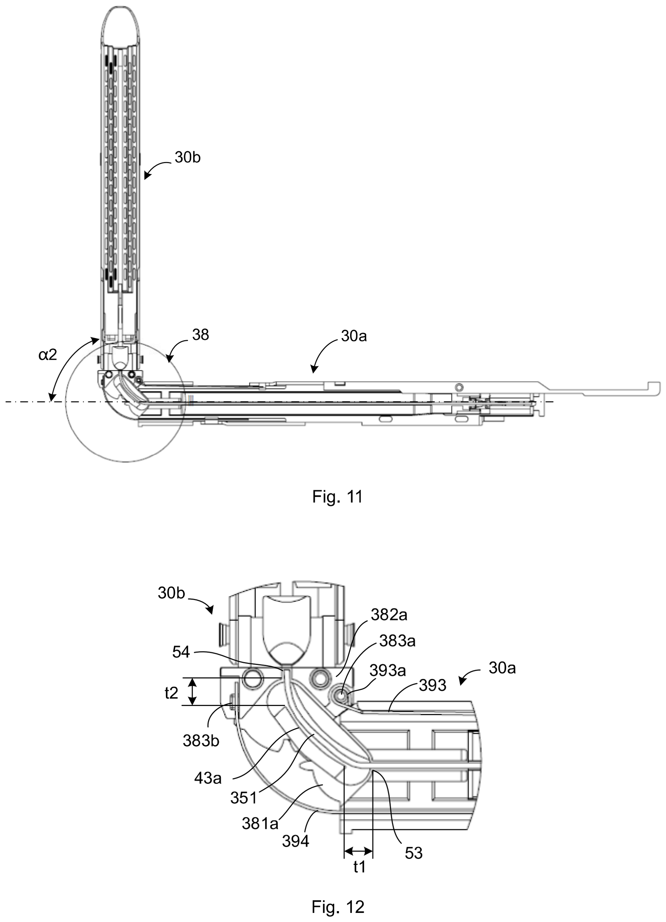

More specifically, a part of the retainer 384 where the first support wall 43a is located is a first support region 384a, and a part of the retainer 384 where the second support wall 43b is located is a second support region 384b. To prevent the retainer 384 from interfering with the firing member 35, the proximal connecting member 381a, or the distal connecting member 382a during operation, the arc length of the first support wall 43a is shorter than the arc length of the second support wall 43b. The proximal end face of the first support region 384a is located distal to the proximal end face of the second support region 384b, and the distal end face of the first support region 384a is located proximal to the distal end face of the second support region 384b. Thus, as shown in Fig. 12, when the distal execution portion 30b is articulated to its maximum articulation angle, certain gaps exist between both ends of the first support wall 43a of the retainer 384 and the proximal connecting member 381a and the distal connecting member 382a. As shown in Fig. 9, a gap between the proximal end of the first support wall 43a and the distal end of the firing channel 53 of the proximal connecting member 381a is t1, and a gap between the distal end of the first support wall 43a and the proximal end of the firing channel 54 of the distal connecting member 382a is t2. As shown in Figs. 9 and 10, when the distal execution portion 30b extends along the longitudinal axis C, the distal edge of the first support wall 43a, the firing channel of the distal execution portion 30b and the inner wall of the firing channel 54 of the distal connecting member 382a are substantially aligned in a straight line. Since the firing beam 351 extends along the firing channel of the proximal body portion 30a to the firing channel of the distal execution portion 30b, the distal edge of the first support wall 43a is located at a position where it can abut against or approach the outer side wall of the firing beam 351, that is, at a position close to a line connecting the axes of the proximal pivot shaft 41 and the distal pivot shaft 42. More specifically, the distance between the proximal edge of the first support wall 43a and the axis of the proximal pivot shaft 41 is less than a first set value, where the first set value is 0.1 mm to 2 mm. Similarly, the proximal end of the first support wall 43a, the inner wall of the firing channel of the proximal body portion 30a and the inner wall of the firing channel 53 of the proximal connecting member 381a are substantially aligned in a straight line. The proximal edge of the first support wall 43a is located at a position where it can abut against or approach the outer side wall of the firing beam 351, namely, at a position close to the line connecting the axes of the proximal pivot shaft 41 and the distal pivot shaft 42. The distance between the distal edge of the first support wall 43a and the axis of the distal pivot shaft 42 is less than a second set value, where the second set value is 0.5 mm to 1 mm. Thus, during the articulation of the distal execution portion 30b, the size of the gap t1 between the proximal end of the first support wall 43a and the distal end of the firing channel of the proximal connecting member 381a remains constant or varies slightly, and the size of the gap t2 between the distal end of the first support wall 43a and the proximal end of the firing channel of the upper distal connecting member 382a also remains constant or varies slightly. This avoids the problem that the firing member 35 cannot be effectively limited due to a large change in the gaps between the retainer 384 and the proximal connecting member 381a and the distal connecting member 382a during the articulation process of the distal execution portion 30b. In an alternative implementation, the firing beam 351 can be effectively limited by further reducing the gap t2 between the distal end of the first support wall 43a and the proximal end of the firing channel 54 of the distal connecting member 382a, and the gap t1 between the proximal end of the first support wall 43a and the distal end of the firing channel 53 of the proximal connecting member 381a. For example, when the distal execution portion 30b extends along the longitudinal axis C, the distal end of the first support wall 43a and the proximal end of the firing channel 54 of the distal connecting member 382a are in a clearance fit, that is, the gap between the two is small; and the proximal end of the first support wall 43a and the distal end of the firing channel 53 of the proximal connecting member 381a are in a clearance fit, that is, the gap between the two is small. This allows the gaps between the retainer 384 and the proximal connecting members 381a and the distal connecting member 382a to be smaller when a first position of the distal execution portion 30b extending along the longitudinal axis C swings to the maximum articulation position.

In the end effector assembly 30 described in the implementation of the present invention, its articulation joint assembly 38 forms the pivot point A of the distal execution portion 30b in such a manner that the proximal connecting member 381a and the distal connecting member 382a mesh with each other, which can realize large-angle articulation in the case of a small articulating radius. Also, the distal connecting member 382a is provided with a articulation driving portion 383a, and it is spaced farther apart from the meshing point (pivot point A) between the proximal connecting members 381a and the distal connecting member 382a. The driving force for articulation is relatively small under the same articulation torque, making it easy to realize the driving of articulation. In addition, since the retainer 384 is pivotally connected to the proximal connecting member 381a and the distal connecting member 382a through two pivot shafts 41, 42, and the center distance between the proximal connecting member 381a and the distal connecting member 382a is constant, the consistency of the firing stroke in the articulated state and the straightened state can be ensured to the greatest extent; for an electric stapler, the formation of the farthest row of staples can be ensured.

To further improve the articulation stability of the distal execution portion 30b, the distal connecting member 382a is provided with two articulation driving portions 383a and 383b. The articulating member 36 is connected to the two articulation driving portions 383a and 383b through a transmission assembly 39. The two articulation driving portions 383a and 383b are located on opposite sides of the longitudinal axis C, respectively. One side of the distal connecting member 382a is subjected to a force toward the distal end, and the other side is subjected to a force toward the proximal end. By arranging the articulation driving portions 383 on both sides of the distal connecting member 382a, the articulation driving force of the articulating member 36 is further reduced, and the articulation stability of the distal connecting member 382a can be better.

With reference to Figs. 4 and 5, the transmission assembly 39 includes: a first rack 391 connected to the articulating member 36, the first rack 391 being mounted on the support main body 34 inside the proximal body portion 30a (see Fig. 3; not shown in Fig. 4); and a first articulation transmission member 393 fixedly connected to the first rack 391, the first articulation transmission member 393 being connected to the first articulation driving portion 383a of the distal connecting member 382a. The transmission assembly 39 further includes: a second rack 392 arranged on the support main body 34, the second rack 392 being spaced apart and opposed to the first rack 391; a second articulation transmission member 394 fixedly connected to the second rack 392, the second articulation transmission member 394 being connected to the second articulation driving portion 383b of the distal connecting member 382a; and two gears 395 located between the first rack 391 and the second rack 392, the two gears 395 being rotatably connected to a gear support frame 396 of the support main body 34. In the implementation as shown in Fig. 4, two gears 395 are arranged to be engaged with the first rack 391 and the second rack 392, respectively. When the articulating member 36 is operated to move toward the distal side, the first rack 391 and the first articulation transmission member 393 are pushed to move toward the distal side. The movement of the gears 395 drives the gears 395 to rotate and causes the second rack 392 to move toward the proximal side, which in turn drives the second articulation transmission member 394 to move toward the proximal side, causing the distal connecting member 382a to articulate.

As an alternative implementation, as shown in Figs. 8 and 9, the first articulation driving portion 383a of the distal connecting member 382a is configured as a pivot structure with its axis direction perpendicular to the clamping face, and the second articulation driving portion 383b is configured as a stud structure with its axis direction parallel to the clamping face. Correspondingly, the distal end of the first articulation transmission member 393 is provided with a connecting sleeve 393a, and the connecting sleeve 393a is sleeved onto the first articulation driving portion 383a through the connecting sleeve 393a; the distal end of the second articulation transmission member 394 is provided with a rivet hole 394a, and the second articulation driving portion 383b engages with the rivet hole 394a to rivet the second articulation transmission member 394 to the distal connecting member 382a. When the distal execution portion 30b is operated to articulate, the articulating radius at the first articulation driving portion 383a (i.e., the articulating radius of the first articulation transmission member 393 connected to the first articulation driving portion 383a when it articulates) is less than the articulating radius at the second articulation driving portion 383b (i.e., the articulating radius of the second articulation transmission member 394 connected to the second articulation driving portion 383b when it articulates).

Since the distal effector assembly 30 described in the implementation of the present invention is mainly operated to be articulated to a large angle toward one side of the longitudinal axis C, the articulating radius of the first articulation driving portion 383a of the distal connecting member 382a is always less than the articulating radius of the second articulation driving portion 383b, causing the deformation of the first articulation transmission member 393 to be greater than the deformation of the second articulation transmission member 394. The first articulation transmission member 393, by using a connecting sleeve 393a to be sleeved on the first articulation driving portion 383a, can improve the connection reliability between the first articulation transmission member 393 and the distal connecting member 382a.

Figs. 8 to 13 show a joint movement process of an end effector assembly 30 provided in an embodiment of the present invention. As shown in Figs. 8 to 10, in this case, the distal execution portion 30b is operated to extend along the direction of the longitudinal axis C. That is, when the distal execution portion 30b and the proximal body portion 30a extend in the same direction, the included angle between the distal execution portion 30b and the longitudinal axis C is 0° or approximately 0°. The first articulation driving portion 383a and the second articulation driving portion 383b of the distal connecting member 382a are each located distal to the pivot point A. Operating the articulation knob 12 of the surgical instrument 100 causes the articulating member 36 to move toward the proximal side, driving the first articulation driving portion 383a and the second articulation driving portion 383b to pivot about the pivot point A, so that the distal execution portion 30b articulates gradually away from the longitudinal axis C, and ultimately reaches a position of the maximum unilateral articulation angle, as shown in Figs. 11 to 13. It can be understood that, during the articulation of the distal execution portion 30b relative to the proximal body portion 30a, the meshing position (i.e., pivot point A) of the proximal connecting member 381a and the distal connecting member 382a does not remain fixed, but changes with the position of the meshing teeth.

In a specific implementation, the articulation joint assembly 38 may use a proximal connecting member 381a and a distal connecting member 382a to achieve a articulating action of the distal execution portion 30b; as an alternative implementation, with reference to Figs. 3 to 5, two proximal connecting members are provided, namely, an upper proximal connecting member 381a and a lower proximal connecting member 381b; and two distal connecting members are also provided, namely an upper distal connecting member 382a and a lower distal connecting member 382b. In this implementation, the upper proximal connecting member 381a extends toward the distal side from the distal end of the first half support main body 34a, and they are fixedly connected in, for example, such a manner that a snap slot is engaged with a snap tab. Similarly, the lower proximal connecting member 381b extends toward the distal side from the distal end of the second half support main body 34b, and the lower proximal connecting member 381b is fixedly connected to the second half support main body 34b in such a manner that a snap slot is engaged with a snap tab. The upper distal connecting member 382a is connected to the staple cartridge assembly 31, and the lower distal connecting member 382b is connected to the staple anvil assembly 32. Providing two sets of proximal connecting members and two sets of distal connecting members further improves the articulation stability of the distal execution portion 30b.

Specifically, the upper distal connecting member 382a is connected to the staple cartridge base 311 through a positioning pin, so that the upper distal connecting member 382a is fixedly connected to the staple cartridge base 311 in an un-articulable manner. The lower distal connecting member 382b is connected to the staple anvil housing 321 through a positioning pin, so that the lower distal connecting member 382b is fixedly connected to the staple anvil housing 321 in an un-articulable manner. Further, the upper distal connecting member 382a is connected to the lower distal connecting member 382b through a positioning pin, so that the upper distal connecting member 382a is fixedly connected to the lower distal connecting member 382b, thereby driving the staple cartridge assembly 31 and the staple anvil assembly 32 to articulate synchronously. It can be understood that the upper distal connecting member 382a and the lower distal connecting member 382b are fixedly connected to the staple cartridge assembly 31 and the staple anvil assembly 32 in an un-articulable manner, whereas the staple cartridge assembly 31 and/or the staple anvil assembly 32 may pivot in closing and opening directions relative to the upper distal connecting member 382a and the lower distal connecting member 382b. The two upper and lower proximal connecting members and distal connecting members are provided, respectively, so that the staple anvil assembly 32 and the staple cartridge assembly 31 of the distal execution portion 30b can be articulated with better synchronization, more uniform articulating action positions, and smooth articulation. Certainly, it can be understood that one proximal connecting member 381a and one distal connecting member 382a may also be provided, which can similarly enable the distal execution portion 30b to articulate about the pivot point A formed by the proximal connecting member 381a and the distal connecting member 382a.

Further, in this implementation, the retainer 384 is accommodated within the mounting space formed between the upper and lower distal connecting members 382a, 382b and the upper and lower proximal connecting members 381a, 381a, preventing the retainer from being exposed to the outside, so as to ensure the movement stability of the firing member 35. Specifically, as shown in Fig. 10, the upper proximal connecting member 381a is provided with a proximal pivot hole 51, and the upper distal connecting member 382a is provided with a distal pivot hole 52. The proximal pivot shaft 41 of the retainer 384 passes through the proximal pivot hole 51 of the upper proximal connecting member 381a, and the distal pivot shaft 42 of the retainer 384 passes through the distal pivot hole 52 of the upper distal connecting member 382a. The end portions of the pivot shafts 41 and 42 are riveted to a first connecting piece 385. The first connecting piece 385 is provided with two connecting holes, and the end portions of the proximal pivot shaft 41 and the distal pivot shaft 42 of the retainer 384 are riveted to the connecting holes of the first connecting piece 385.

The articulation joint assembly 38 further includes a second connecting piece 386. As shown in Fig. 3, the lower proximal connecting member 381b and the lower distal connecting member 382b are riveted to the second connecting piece 386 through rotating shafts, respectively. The second connecting piece 386 is provided with two connecting holes, and the end portions of the rotating shafts on the lower proximal connecting member 381b and the lower distal connecting member 382b are riveted to the connecting holes of the second connecting piece 386.

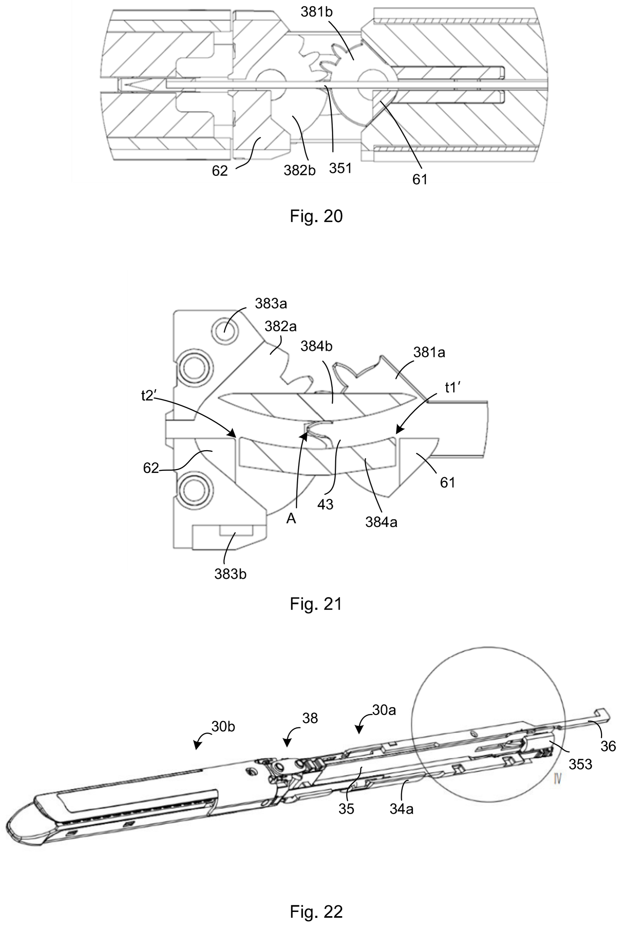

To better accommodate the firing member 35 within the articulation joint assembly 38 and prevent the firing beam 351 of the firing member 35 from popping out of the joint or stacking up, in an alternative implementation, the lower proximal connecting member 381b is further provided with a first limiting protrusion 61 on a side surface facing the retainer 384. A part of the side surface of the first limiting protrusion 61 further limits the articulation position of the firing member 35. The first limiting protrusion 61 is opposite to the proximal end face of the first support region 384a of the retainer 384 to define a first gap t1' therebetween. Specifically, t1'<t1. When the distal execution portion 30b swings from a first position extending along the longitudinal axis to a maximum articulation position, the first gap t1' remains unchanged or changes slightly. As shown in Fig. 21, the first limiting protrusion 61 has a first side surface arranged opposite to the proximal end face of the first support region 384a and a second side surface arranged opposite to the firing member 35. The first side surface is arranged perpendicular to the second side surface. In other alternative implementations, the included angle between the first side surface and the second side surface of the first limiting protrusion 61 is between 80° and 100°, depending on the maximum articulation angle of the distal execution portion 30b. With the provision of the above first limiting protrusion 61, during the process of the end effector assembly 30b changing from a straightened state to a maximum articulation angle, the gap t1' between the retainer 384 and the first limiting protrusion 61 on the proximal side of the articulation joint assembly 38 is always small. The firing beam 351 is reliably articulated under the position-limiting action of the retainer 384 and the first limiting protrusion 61.

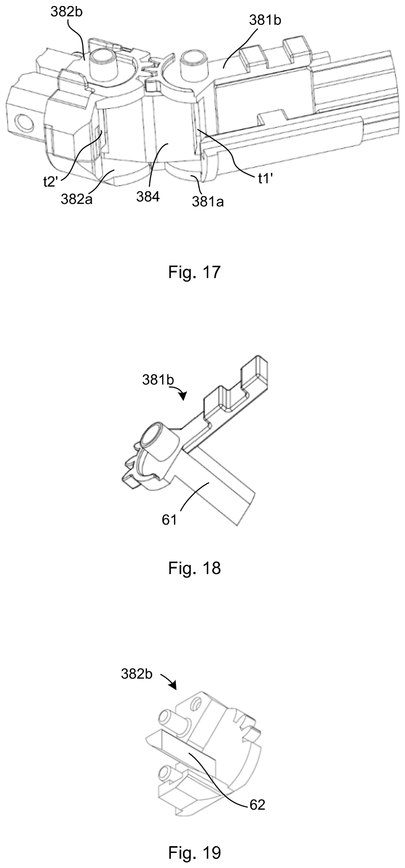

Correspondingly, as shown in Fig. 19, the lower distal connecting member 382b is provided with a second limiting protrusion 62 on a side surface facing the retainer 384. A part of the side surface of the second limiting protrusion 62 further limits the articulation position of the firing member 35. The second limiting protrusion 62 is opposite to the distal end face of the first support region 384a of the retainer 384 to define a second gap t2' therebetween. Specifically, t2'<t2. When the distal execution portion 30b swings from a first position extending along the longitudinal axis to a maximum articulation position, the second gap t2' remains unchanged or changes slightly. As shown in Fig. 21, the second limiting protrusion 62 also has a first side surface arranged opposite to the first support region 384a and a second side surface arranged opposite to the firing member 35. The first side surface is arranged perpendicular to the second side surface. In other alternative implementations, the included angle between the first side surface and the second side surface is between 80° and 100°, depending on the maximum articulation angle of the distal execution portion 30b. With the provision of the above second limiting protrusion 62, during the process of the end effector assembly changing from the straightened state to the maximum articulation angle, the gap t2' between the retainer 384 and the first limiting protrusion 61 on the distal side of the articulation joint assembly 38 is always small, and the firing beam is reliably articulated under the position-limiting action of the retainer 384 and the second limiting protrusion 62.

It can be understood that, in other alternative implementations, the first limiting protrusion 61 may also be provided on the proximal connecting member 381a, and the second limiting protrusion 62 may also be arranged on the distal connecting member 382a.

Further, as shown in Fig. 21, when the distal execution portion 30b extends along the direction of the longitudinal axis, the second side surfaces of the first limiting protrusion 61 and the second limiting protrusion 62 are parallel to the longitudinal axis. The first side surface of the first limiting protrusion 61 is parallel to the proximal end face of the first support region 384a of the retainer 384, and the first side surface of the second limiting protrusion 62 is parallel to the distal end face of the first support region 384a of the retainer 384.

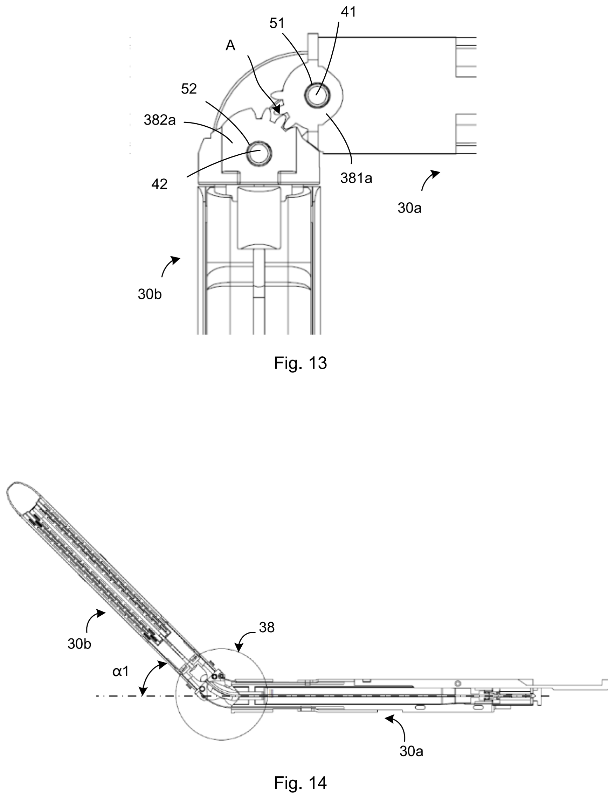

To enable the end effector assembly 30 described in the embodiment of the present invention to be adapted to a main body portion (including a handle assembly and an elongated body assembly) of a surgical instrument 100 in the prior art, that is, to enable the end effector assembly 30 described in the embodiment of the present invention to be adapted to the main body portion (including the handle assembly and the elongated body assembly) of the same surgical instrument 100 like end effector assemblies of the same specifications and dimensions in existing designs, in an alternative implementation, the end effector assembly 30 is initially preset with a certain articulation angle before being loaded onto the surgical instrument. For example, as shown in Fig. 14, when the end effector assembly 30 is in a ready-to-load position, the distal execution portion 30b forms a predetermined first included angle α₁ with respect to the longitudinal axis C.

Specifically, after the end effector assembly 30 in the ready-to-load position is mounted on the elongated body assembly 20 of the surgical instrument 100, the articulation knob 12 of the handle assembly 10 is operated to move the articulating member 36 toward the proximal side by a first distance. Correspondingly, the distal execution portion 30b of the end effector assembly 30, having already been articulated at the first included angle α₁, is further articulated away from the longitudinal axis C to a second included angle α₂, as shown in Figs. 11 and 12. Similarly, after the end effector assembly 30 in the ready-to-load position is mounted on the elongated body assembly 20 of the surgical instrument 100, the articulation knob 12 of the handle assembly 10 is operated to move the articulating member 36 toward the distal side by a second distance. Correspondingly, the distal execution portion 30b of the end effector assembly 30, having already been articulated at the first included angle α₁, is further pivoted toward the longitudinal axis C, ultimately forming an angle of 0° or approximately 0° with respect to the longitudinal axis C, as shown in Figs. 8 and 9. The first distance and the second distance may be the same or different.

When the end effector assembly 30 described in the embodiment of the present invention is adapted to the surgical instrument 100 in the prior art, the first distance may correspond to the distance the articulating member 36 moves when the end effector assembly 30 is articulated to the right to its maximum angle; the second distance may correspond to the distance the articulating member 36 moves when the end effector assembly 30 is articulated to the left to its maximum angle. This configuration of the end effector assembly 30 further improves the versatility and adaptability of the end effector assembly described in the embodiment of the present invention.

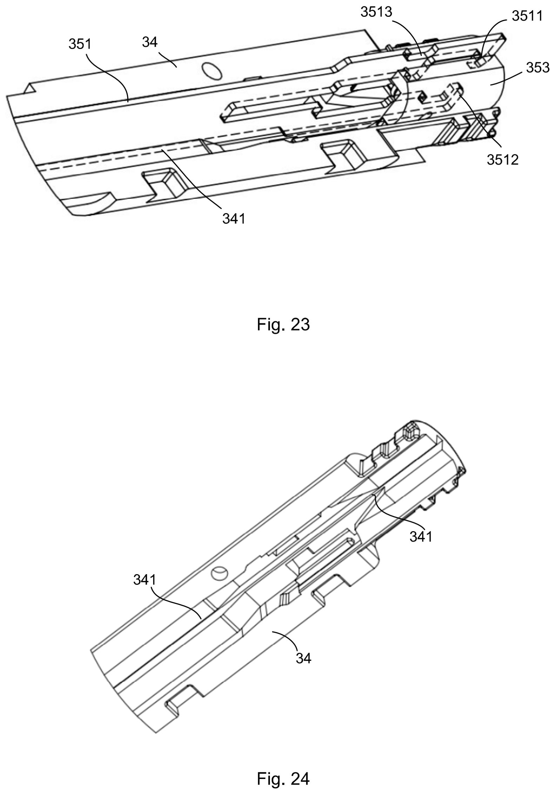

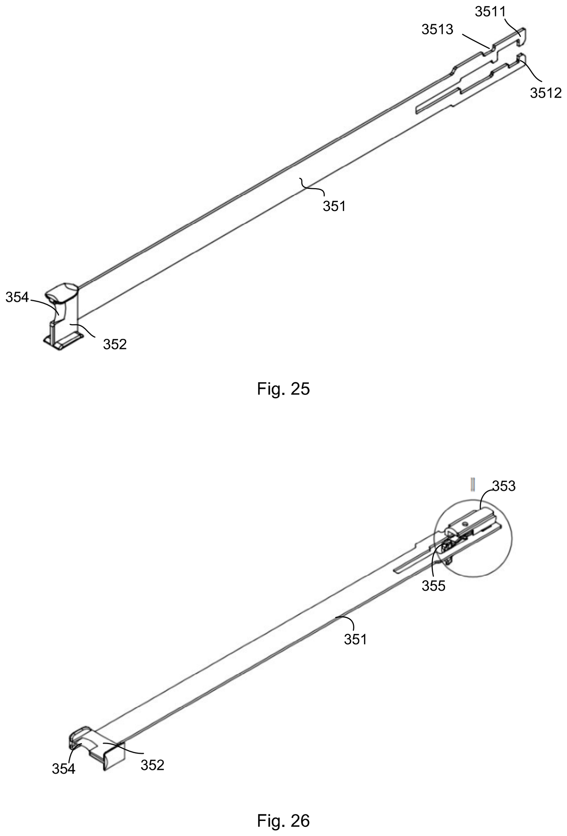

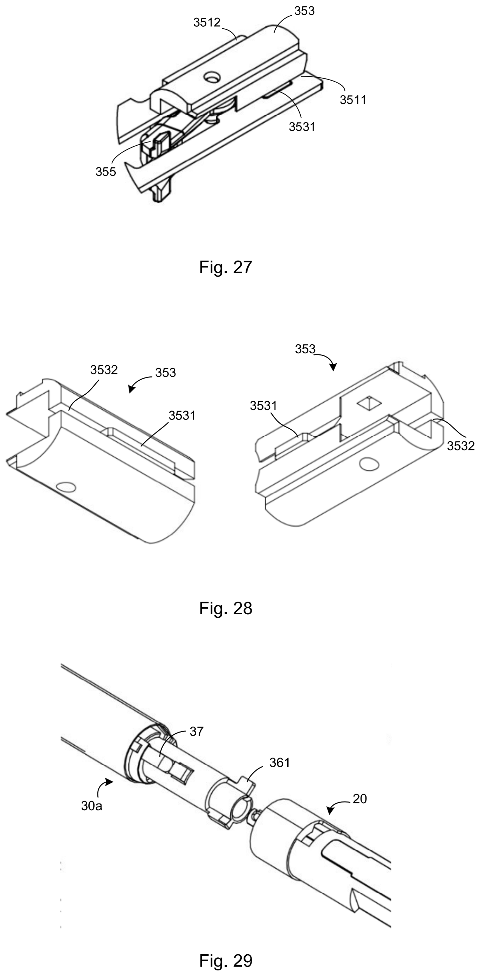

Further, in the end effector assembly 30 described in the implementation of the present invention, a guide slot 341 (see Figs. 23 and 24) for slidably receiving the firing member 35 is defined between the first half support main body 34a and the second half support main body 34b, thereby forming a sliding channel. The firing member 35 includes an elongated firing beam 351 slidably connected to the guide slot 341. The guide slot 341 extends along the direction of the longitudinal axis and penetrates the extension regions of the first half support main body 34a and the second half support main body 34b, so that both the proximal and distal ends of the support main body 34 can positionally limit the firing beam 351. The proximal end of the firing beam 351 is hooked and connected to the firing connecting member 353. Specifically, as shown in Figs. 22 and 23, the firing connecting member 353 is formed into a sleeve structure with an opening. The proximal side of the firing beam 351 is configured as a structure with an opening, and its proximal end portion has a first hook portion 3511 and a second hook portion 3512 arranged opposite to each other. The firing connecting member 353 is provided with a firing connecting portion 3531, and the first hook portion 3511 and the second hook portion 3512 are hooked on opposite sides of the firing connecting portion 3531. The firing connecting member 353 is provided with a guide track 3532 at the distal end of the firing connecting portion 3531. The guide track 3532 extends along the direction of the longitudinal axis and is suitable for limiting the position of the two side surfaces of the firing beam 351 perpendicular to the longitudinal axis. For example, when the firing beam 351 is configured as a stack of sheets, the guide track 3532 is configured to engage with the sheet-like surfaces on both sides of the firing beam 351 to limit its position and prevent the stacked sheet-like firing beam 351 from shifting when turning. As shown in Figs. 26 and 27, the firing connecting member 353 is further provided with a safety mechanism 355 for preventing double firing of the firing member 35. For the specific structure of the safety mechanism 355, please refer to a detailed description in Chines Patent Application No. CN202210770831.1, which is incorporated by reference in its entirety and will not be repeated here.

Further, as shown in Fig. 29, the proximal body portion 30a further includes a locking member 37 for making the articulating member 36 and the firing member 35 be in initial positions when the end effector assembly 30 is in the ready-to-load position. Specifically, as shown in Fig. 12, the locking member 37 includes a first locking portion 371 and a second locking portion 372. When the end effector assembly 30 is in the ready-to-load position, the first locking portion 371 engages with the articulating member 36 to lock the articulating member 36, and the second locking portion 372 engages with the firing member 35 to lock the firing member 35. By adopting this locking member 37, both the articulating member 36 and the firing member 35 can be locked simultaneously.

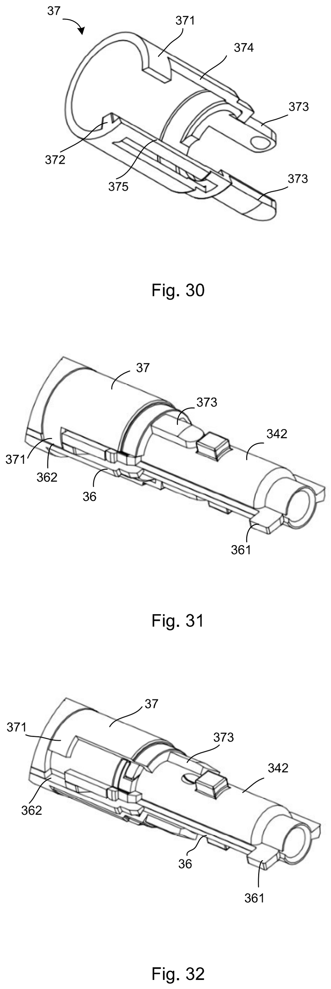

The locking member 37 is rotatably sleeved on the engagement portion 34c located on the proximal side of the support main body 34, and may be switched between a locked position and an unlocked position. When the locking member 37 is rotated to the locked position, as shown in Fig. 31, the first locking portion 371 engages with the articulating member 36 to lock, and the second locking portion 372 engages with the firing member 35 to lock. At this time, the end effector assembly 30 is in the ready-to-load position. When the locking member 37 is rotated to the unlocked position, as shown in Fig. 32, the first locking portion 371 disengages from the articulating member 36, and the second locking portion 372 disengages from the firing member 35. The articulating member 36 is unlocked from the firing member 35, and may slide relative to the support main body 34.

The locking member 37 is engaged in a locked position with the articulating member 36 and the firing member 35 by means of rotational insertion. Specifically, as shown in Fig. 12, the locking member 37 is configured as a semi-annular sleeve with a notch. The semi-annular sleeve has a first wall surface 374 and a second wall surface 375 that are arranged opposite to each other and form the notch. The first locking portion 371 is configured as a first protrusion extending circumferentially along the side wall (i.e., the first wall surface 374 or the second wall surface 375) of the locking member 37 that forms the notch. The second locking portion 372 is configured as a second protrusion extending inwardly along the inner wall of the locking member 37. Correspondingly, as shown in Figs. 31 and 32, the articulating member 36 is provided with a articulation locking slot 362. When the locking member 37 is rotated to the locked position, the first protrusion of the locking member 37 is inserted into the articulation locking slot 362 of the articulating member 36 to lock the articulating member 36. The firing beam 351 is provided with a firing locking slot 3513 (with reference to Fig. 25). When the locking member 37 is rotated to the locked position, the second protrusion of the locking member 37 is inserted into the firing locking slot 3513 of the firing member 35 to simultaneously lock the firing member 35.

As shown in Figs. 30 to 32, the locking member 37 further includes an unlocking driving portion 373. When the end effector assembly 30 is in a loading position, the unlocking driving portion 373 is subjected to a circumferential force of the driving protrusion within the elongated body assembly 20, driving the locking member 37 to rotate around the support main body 34 and disengage from the articulating member 36 and the firing member 35. The unlocking driving portion 373 is configured as an extension piece extending toward the proximal end along the proximal end face of the semi-annular sleeve. Also, to improve the rotational stability of the locking member 37, two unlocking driving portions 373 are symmetrically arranged along the circumference of the locking member 37. Correspondingly, two driving protrusions for driving the unlocking driving portions 373 are arranged on the inner wall of the elongated main body of the elongated body assembly 20.

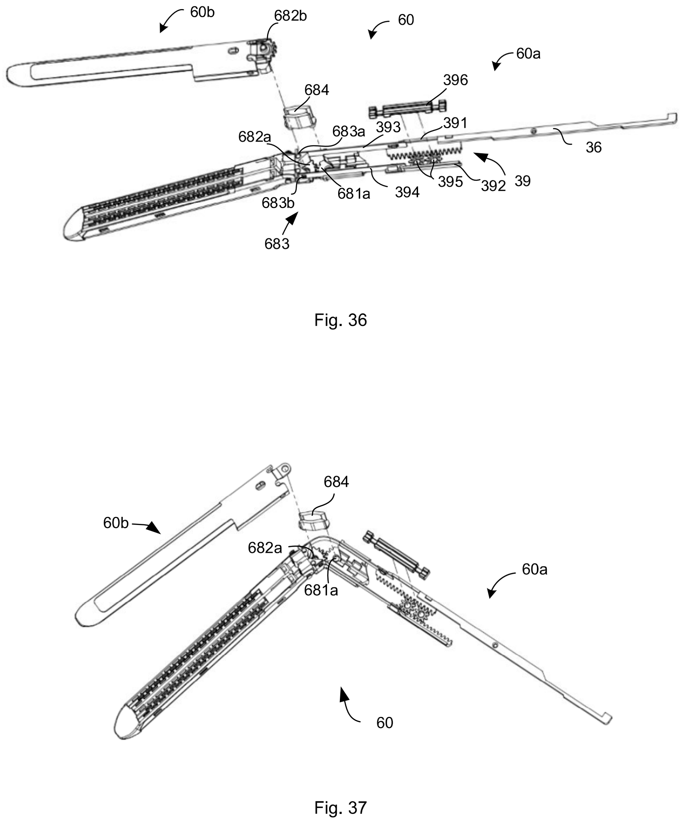

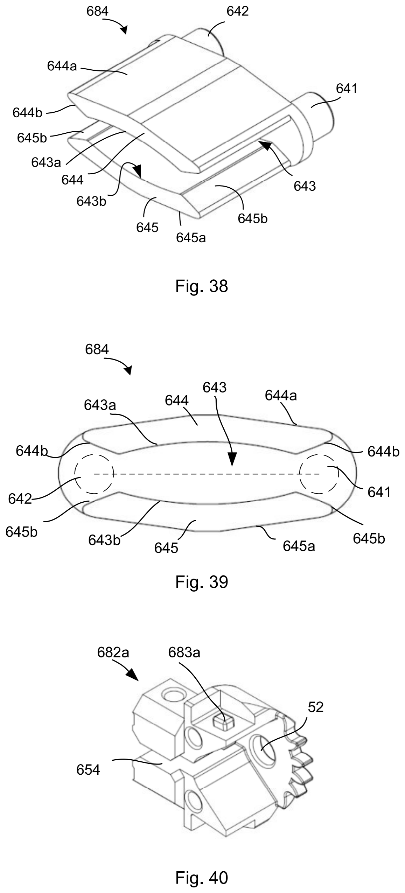

Figs. 36 to 47 show an end effector assembly 60 described in another implementation of the present invention. It can be understood that a part of the structure of the end effector assembly 60 described in this embodiment adopts the same design as the end effector assembly 30. Therefore, the structure or components that adopt the same design as the end effector assembly 30 will be described using the reference signs in the end effector assembly 30. In this implementation, the end effector assembly 60 includes a retainer 684, and the retaining channel 643 of the retainer 684 is configured as an arc structure as shown in Fig. 38 or 39. The curved channel is formed by a first support wall 643a and a second support wall 643b located on both sides of the firing beam 351. The first support wall 643a and the second support wall 643b are curved in opposite directions, and the first support wall 643a and second support wall 643b are each arc-shaped with a direction of curvature that is convex outward. When the distal execution portion 60b of the end effector assembly 60 is articulated relative to the proximal body portion 60a, the first support wall 643a or the second support wall 643b supports the outer arc articulated surface of the firing member 35.

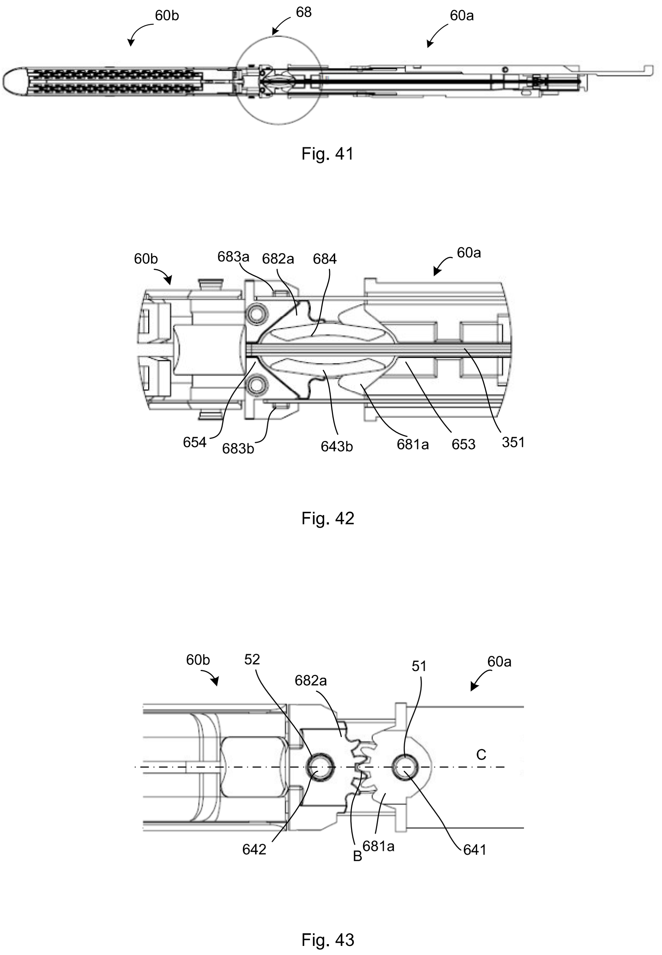

As shown in Fig. 42, similar to the previous embodiment, the proximal connecting member 681a of the end effector assembly 60 includes a firing channel 653, with a proximal end opposite to the firing channel of the proximal body portion 60a and a distal end opposite to the retaining channel 643 of the retainer 684. Correspondingly, the distal connecting member 682a also has a firing channel 654 with a distal end opposite to the firing channel of the distal body portion 60a and a proximal end opposite to the retaining channel 643 of the retainer 684. When the distal execution portion 60b extends along the direction of the longitudinal axis C, the firing channel 653 of the proximal connecting member 681a and the firing channel 654 of the distal connecting member 682a extend in the same direction and are positioned opposite to each other, which is suitable for the firing beam 351 to extend in a straight line.

With reference to Figs. 38 and 39, a part of the retainer 684 where the first support wall 643a is located is a first support region 644. The outer side wall of the first support region 644 is a first outer arc wall 644a. The first outer arc wall 644a has a direction of curvature same as that of the first support wall 643a and an arc length greater than that of the first support wall 643a. The first outer arc wall 644a and the first support wall 643a are transitioned at both ends by means of first transition walls 644b, respectively. A part of the retainer 684 where the second support wall 643b is located is a second support region 645. The outer side wall of the second support region 645 is a second outer arc wall 645a. The second outer arc wall 645a has a direction of curvature same as that of the second support wall 643b and an arc length greater than that of the second support wall 643b.The second outer arc wall 645a and the second support wall 643b are transitioned at both ends by means of second transition walls 645b, respectively.

The end effector assembly 60 shown in Figs. 41 and 42 is in a non-articulated position, that is, the distal execution portion 60b extends along the longitudinal axis C. In this case, the distal edges of the first support wall 643a and the second support wall 643b and the inner walls of the firing channel of the distal execution portion 60b and the firing channel 654 of the distal connecting member 682a are substantially aligned in a straight line. The proximal ends of the first support wall 643a and the second support wall 643b and the inner walls of the firing channel of the proximal body portion 60a and the firing channel 653 of the proximal connecting member 681a are substantially aligned in a straight line. This enables the firing beam 351 to be limited and protected by the first support wall 643a and the second support wall 643b of the retainer 684 in a straightened state.

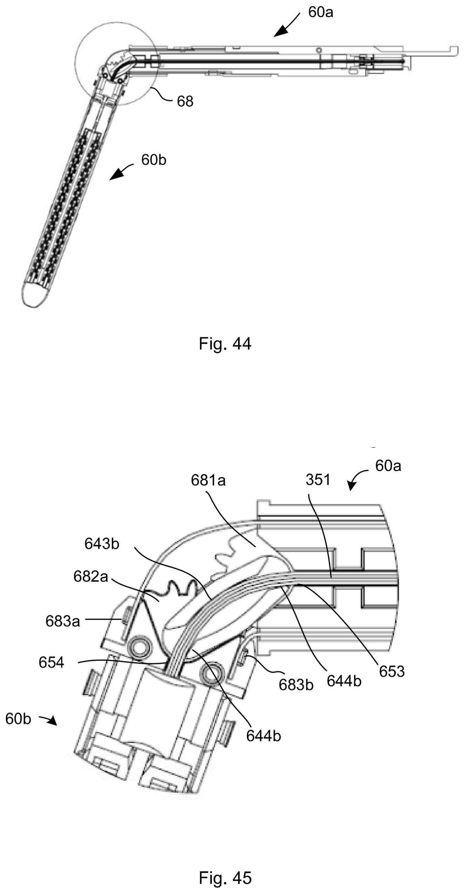

With reference to Figs. 44 and 45, when the distal execution portion 60b is articulated to a maximum articulation angle in a first direction relative to the proximal body portion 60a, the first transition wall 644b on the proximal side of the retainer 684 supports and limits the proximal inner arc surface position of the firing member 35, the first transition wall 644b on the distal side of the retainer 684 supports and limits the distal inner arc surface position of the firing member 35, and the second support wall 643b of the retainer 684 limits the maximum articulation outer arc surface position of the firing member 35. When the distal execution portion 60b is articulated to a maximum articulation angle in a second direction (opposite to the first direction) relative to the proximal body portion 60a, the second transition wall 645b on the proximal side of the retainer 684 limits the proximal inner arc surface position of the firing member 35, the second transition wall 645b on the distal side of the retainer 684 limits the distal inner arc surface position of the firing member 35, and the first support wall 643a of the retainer 684 limits the maximum articulation outer arc surface position of the firing member 35. The limiting and protection of the retainer 684 at the three positions allow the firing member 35 to articulate at a preset articulation position, preventing it from popping out of the articulation joint assembly 68.

Further, with reference to Fig. 45, a portion of the firing member 35 that engages with the first transition wall 644b or the second transition wall 645b on the proximal side is a curved transition region on the proximal side of the firing member 35, and a portion of the firing member 35 that engages with the first transition wall 644b or the second transition wall 645b on the distal side is a curved transition region on the distal side of the firing member 35. To cause the first transition wall 644b or the second transition wall 645b of the retainer 684 to better engage with the firing member 35 to limit the position of the firing member 35, a portion of the first transition wall 644b or the second transition wall 645b of the retainer 684 is a straight/planar surface structure, which can increase the contact area with the side wall of the firing beam 351 of the firing member 35, thereby achieving a better limiting effect.

With reference to Fig. 45, when the distal execution portion 60b is articulated to the maximum articulation angle relative to the proximal body portion 60a, the proximal end of the first transition wall 644b or the second transition wall 645b located on the proximal side substantially abuts against the inner wall of the firing channel 653 of the proximal connecting member 681a to limit the articulation of the firing member 35, and the distal end of the first transition wall 644b or the second transition wall 645b located on the distal side substantially abuts against the inner wall of the firing channel 654 of the distal connecting member 682a to limit the articulation of the firing member 35, so that the firing beam 351 can be limited and protected by the retainer 684 in the part of the region of the articulation joint assembly 68.

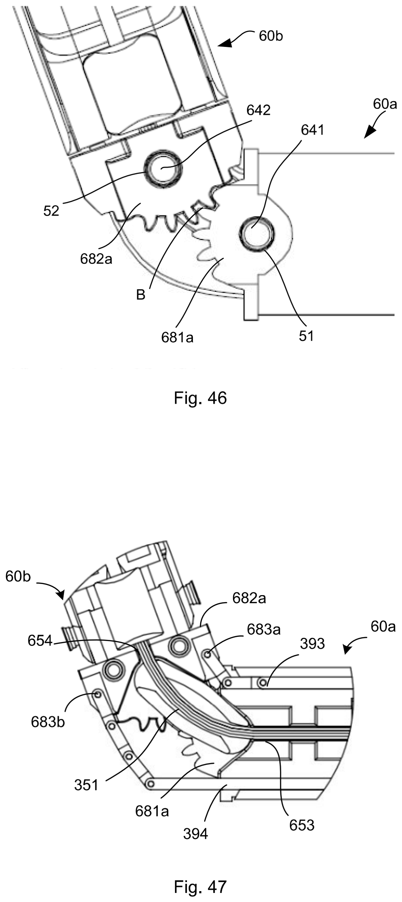

In the end effector assembly 60 described in the implementation of the present invention, its articulation joint assembly68 forms the pivot point B of the distal execution portion 60b in such a manner that the proximal connecting member 681a and the distal connecting member 682a mesh with each other (as shown in Figs. 43 and 46), which can realize large-angle articulation in the case of a small articulating radius. Similar to the previous embodiment, in this embodiment, the distal connecting member 682a is also provided with a articulation driving portion 683a, and it is spaced farther apart from the meshing point (pivot point B) between the proximal connecting members 681a and the distal connecting member 682a. The driving force for articulation is relatively small under the same articulation torque, making it easy to realize the driving of articulation. In addition, since the retainer 684 is connected to the proximal connecting member 681a and the distal connecting member 682a through two pivot shafts, and the center distance between the proximal connecting member 681a and the distal connecting member 682a is constant, the consistency of the firing stroke in the articulated state and the straightened state can be ensured to the greatest extent; for an electric stapler, the formation of the farthest row of staples can be ensured.

It can be understood that the end effector assembly 60 described in this embodiment adopts the same structure as the transmission assembly 39 of the end effector assembly 30 to achieve the operation of articulation the proximal body portion 60a, which will not be repeated here. Moreover, in this embodiment, to further improve the articulation stability of the distal execution portion 60b, as shown in Figs. 42 and 45, the distal connecting member 682a may also be provided with two articulation driving portions 683: a first articulation driving portion 683a and a second articulation driving portion 683b. The articulating member 36 is separately connected to the first articulation driving portion 683a and the second articulation driving portion 683b through the transmission assembly 39. The first articulation driving portion 683a and the second articulation driving portion 683b are located on opposite sides of the longitudinal axis C, respectively. One side of the distal connecting member 682a is subjected to a force toward the distal end, and the other side is subjected to a force toward the proximal end. By providing the articulation driving portions 683 on both sides of the distal connecting member 682a, the articulation driving force of the articulating member 36 is further reduced, and the articulation stability of the distal connecting member 682a can be better.

In an implementation, as shown in Figs. 40, 42, and 45, the two articulation driving portions 683a and 683b of the distal connecting member 682a are driving shaft structures whose axial directions are perpendicular to the clamping face. The distal ends of the first articulation transmission member 393 and the second articulation transmission member 394 are each provided with a rivet hole. The first articulation driving portion 683a and the second articulation driving portion 683a engage with the rivet holes to rivet the first articulation transmission member 393 and the second articulation transmission member 394 to the distal connecting member 682a. In other alternative implementations, the articulation driving portions 683a, 683b of the distal connecting member 682a are driving shaft structures whose axial directions are parallel to the clamping face. The first articulation transmission member 393 and/or the second articulation transmission member 394 are provided with connecting sleeves. The first articulation transmission member 393 and the second articulation transmission member 394 are connected to the distal connecting member 682a in such a manner that the connecting sleeves are sleeved to the driving shafts.

As an alternative implementation, on the basis of the above embodiment, Figs. 33 and 47 show another structural form of the first articulation transmission member 393 and the second articulation transmission member 394, respectively. Specifically, the first articulation transmission member 393 and the second articulation transmission member 394 are configured in a chain form formed by multiple sections of pivotally connected rods, and can articulate accordingly when the distal execution portions 30b, 60b are articulated at a large angle relative to the proximal body portions 30a, 60a. The chain-form structure enables each rod to rotate between the pivot shafts, enabling it to adapt to a larger articulation deformation amount, so that the problems such as breakage caused by exceeding the elastic deformation of elastic materials at large articulation angles can be avoided.

It can be understood that, similar to the aforementioned embodiment, in this embodiment, the articulation joint assembly68 may be provided in the form of a proximal connecting member 681a and a distal connecting member 682a, or in the form of two proximal connecting members and/or two distal connecting members, which will not be repeated here.

Next, a joint movement process of the end effector assembly 60 according to an embodiment of the present invention will be described in detail with reference to Figs. 41 to 46. As shown in Figs. 41 to 43, when the distal execution portion 60b extends along the direction of the longitudinal axis C, that is, when the distal execution portion 60b and the proximal body portion 60a extend in the same direction, the included angle between the distal execution portion 60b and the longitudinal axis C is 0° or approximately 0°. That is, the end effector assembly 60 is in an initial state/position ready to load or unused (hereinafter referred to as a ready-to-load position). The first articulation driving portion 683a and the second articulation driving portion 683b of the distal connecting member 682a are each located distal to the pivot point B. Operating the articulation knob 12 of the surgical instrument 100 causes the articulating member 36 to move toward the proximal side, driving the first articulation driving portion 683a and the second articulation driving portion 683b to pivot about the pivot point B, so that the distal execution portion 60b articulates in a first direction gradually away from the longitudinal axis C, and ultimately reaches a position/state with the maximum articulation angle in the first direction, as shown in Figs. 44 to 46. Operating the articulation knob 12 of the surgical instrument 100 causes the articulating member 36 to move toward the distal side, driving the first articulation driving portion 683a and the second articulation driving portion 683b to pivot about the pivot point B, so that the distal execution portion 60b articulates in a second direction (the opposite direction of the first direction) gradually away from the longitudinal axis C and ultimately reaches a position/state with the maximum articulation angle in the second direction (not shown). The position/state with the maximum articulation angle in the second direction is mirror-symmetrical to the position/state with the maximum articulation angle in the first direction, with the longitudinal axis C as an axis of symmetry.

Obviously, the above embodiments are merely examples for clarity of explanation and are not intended to limit the implementations. For those of ordinary skill in the art, on the basis of the above description, other variations or changes in different forms can also be made. It is not necessary and impossible to exhaustively list all implementations here. Obvious variations or changes arising therefrom remain within the scope of protection of the present invention.

## Claims

1. An end effector assembly of a surgical instrument, comprising:
a proximal body portion defining a longitudinal axis and comprising an articulating member and a firing member;
a distal execution portion comprising a staple cartridge assembly and a staple anvil assembly, wherein opposing surfaces of the staple cartridge assembly and the staple anvil assembly form clamping faces for clamping tissue, and the distal execution portion is pivotally engaged to the proximal body portion through an articulation joint assembly, **characterized in that** the articulation joint assembly comprises:
a proximal connecting member fixed to a distal end of the proximal body portion;
a distal connecting member fixed to a proximal end of the distal execution portion, wherein the distal connecting member is engaged with the proximal connecting member, and the distal connecting member is provided with at least one articulation driving portion that is actuated by the articulating member to move so as to articulate the distal execution portion relative to the proximal body portion; and
a retainer pivotally connected to the proximal connecting member and the distal connecting member respectively, wherein the retainer is provided with a retaining channel for receiving the firing member.

2. The end effector assembly according to claim 1, **characterized in that** a distal end of the proximal connecting member has a toothed portion, and a proximal end of the distal connecting member has a toothed portion that is engaged with the proximal connecting member; and a position in which the distal connecting member meshes with the proximal connecting member forms a pivot point of the distal execution portion, and when the distal execution portion extends along the direction of the longitudinal axis, the articulation driving portion is arranged so as not to overlap with the longitudinal axis.

3. The end effector assembly according to claim 1 or 2, **characterized in that** the retainer is provided with a proximal pivot shaft and a distal pivot shaft respectively; the proximal pivot shaft of the retainer is pivotally engaged with a proximal pivot hole of the proximal connecting member, and the distal pivot shaft of the retainer is pivotally engaged with a distal pivot hole of the distal connecting member; and a line connecting axes of the proximal pivot shaft and the distal pivot shaft is located in the retaining channel.

4. The end effector assembly according to any one of claims 1 to 3, **characterized in that** the articulation joint assembly further comprises a first connecting piece provided with two connecting holes, and a proximal pivot shaft and a distal pivot shaft of the retainer respectively pass through a proximal pivot hole of the proximal connecting member and a distal pivot hole of the distal connecting member and are riveted to the connecting holes of the connecting member.

5. The end effector assembly according to any one of claims 1 to 4, **characterized in that** the distal connecting member is provided with a first articulation driving portion and a second articulation driving portion, and the articulating member is connected to the first articulation driving portion and the second articulation driving portion respectively through a transmission assembly.

6. The end effector assembly according to claim 5, **characterized in that** the transmission assembly comprises:
a first rack and a first articulation transmission member fixedly connected thereto, wherein a proximal end of the first rack is engaged with the articulating member, and the first articulation transmission member is engaged with one of the articulation driving portions of the distal connecting member;
a second rack and a second articulation transmission member fixedly connected thereto, wherein the second rack is arranged opposite to the first rack with a spacing therebetween, and the second articulation transmission member is engaged with the other one of the articulation driving portions of the distal connecting member; and
at least one gear arranged between the first rack and the second rack and being respectively engaged with the first rack and the second rack.

7. The end effector assembly according to claim 6, **characterized in that** the first articulation driving portion and the second articulation driving portion are configured as pivot pins fixed to the distal connecting member, and the first articulation transmission member or the second articulation transmission member is riveted or sleeved on the respective pivot pin.

8. The end effector assembly according to claim 6, **characterized in that** the first articulation driving portion of the distal connecting member is configured as a pivot pin with an axial direction perpendicular to the clamping face, and a distal end of the first articulation transmission member is provided with a connecting sleeve, and the first articulation transmission member is sleeved on the first articulation driving portion through the connecting sleeve.

9. The end effector assembly according to claim 6, **characterized in that** the second articulation driving portion of the distal connecting member is configured as a stud with an axial direction parallel to the clamping face, a distal end of the second articulation transmission member is provided with a rivet hole, and the second articulation driving portion is connected to the second articulation transmission member through the rivet hole.

10. The end effector assembly according to any one of claims 1 to 9, **characterized in that** the retaining channel is a unidirectional curved channel, in which a first support wall and a second support wall defining the retaining channel are curved in the same direction, and when the firing member is articulated to a maximum angle position, the first support wall is adapted to support an outer side wall of the firing member, and the second support wall is adapted to support an inner side wall of the firing member.

11. The end effector assembly according to claim 10, **characterized in that** an arc length of the first support wall is less than an arc length of the second support wall.

12. The end effector assembly according to claim 11, **characterized in that** a portion of the retainer where the first support wall is located is a first support region, and a portion of the retainer where the second support wall is located is a second support region, wherein a proximal end face of the first support region is located distal to a proximal end face of the second support region, and a distal end face of the first support region is located proximal to a distal end face of the second support region.

13. The end effector assembly according to claim 10, **characterized in that** when the distal execution portion extends along the direction of the longitudinal axis, a distal edge of the first support wall is substantially aligned with an inner wall of a firing channel of the distal execution portion; and a proximal end of the first support wall is substantially aligned with an inner wall of a channel supporting relative sliding of the firing member in the proximal body portion

14. The end effector assembly according to claim 13, **characterized in that** when the distal execution portion extends along the direction of the longitudinal axis, a distal end of the first support wall is clearance-fitted with a proximal end of a firing channel of the distal connecting member, and a proximal end of the first support wall is clearance-fitted with a distal end of a firing channel of the proximal connecting member.

15. The end effector assembly according to any one of claims 12 to 14, **characterized in that** the proximal connecting member is provided with a first limiting protrusion on a side facing the retainer, the first limiting protrusion being opposite to a proximal end face of the first support region with a first gap therebetween; and the distal connecting member is provided with a second limiting protrusion on a side facing the retainer, the second limiting protrusion being opposite to a distal end face of the first support region with a second gap therebetween.

16. The end effector assembly according to claim 15, **characterized in that** the first limiting protrusion and the second limiting protrusion each have a first side surface arranged opposite to the first support region and a second side surface arranged opposite to the firing member, and an included angle between the first side surface and the second side surface is between 80° and 100°.

17. The end effector assembly according to claim 16, **characterized in that** when the distal execution portion extends along the direction of the longitudinal axis, the second side surfaces of the first limiting protrusion and the second limiting protrusion are parallel to the longitudinal axis.

18. The end effector assembly according to claim 16 or 17, **characterized in that** when the distal execution portion extends along the direction of the longitudinal axis, the first side surface of the first limiting protrusion is arranged parallel to the proximal end face of the first support region of the retainer, and the first side surface of the second limiting protrusion is parallel to the distal end face of the first support region of the retainer.

19. The end effector according to any one of claims 1 to 9, **characterized in that** the retaining channel is a bidirectional curved channel, in which a first support wall and a second support wall defining the retaining channel are each arc-shaped with a direction of curvature that is convex outward, and when the distal execution portion is articulated relative to the proximal body portion, the first support wall or the second support wall supports an outer arc articulated surface of the firing member.

20. The end effector assembly according to claim 19, **characterized in that** a portion of the retainer where the first support wall is located is a first support region, and an outer side wall of the first support region is a first outer arc wall, wherein the first outer arc wall has a direction of curvature same as that of the first support wall and an arc length greater than that of the first support wall, and the first outer arc wall and the first support wall are transitioned at both ends through first transition walls, respectively; and
a portion of the retainer where the second support wall is located is a second support region, and an outer side wall of the second support region is a second outer arc wall, wherein the second outer arc wall has a direction of curvature same as that of the second support wall and an arc length greater than that of the second support wall, and the second outer arc wall and the second support wall are transitioned at both ends through second transition walls, respectively.

21. The end effector assembly according to claim 20, **characterized in that** when the distal execution portion is articulated to a maximum articulation angle relative to the proximal body portion, the first transition wall or the second transition wall on a proximal side of the retainer limits a proximal inner arc surface position of the firing member, and the first transition wall or the second transition wall on a distal side of the retainer limits a distal inner arc surface position of the firing member.

22. The end effector assembly according to claim 20 or 21, **characterized in that** a portion of the firing member that engages with the first transition wall or the second transition wall on the proximal side is a curved transition region on a proximal side of the firing member, and a portion of the firing member that engages with the first transition wall or the second transition wall on the distal side is a curved transition region on a distal side of the firing member.

23. The end effector assembly according to any one of claims 20 to 22, **characterized in that** when the distal execution portion is articulated to a maximum articulation angle relative to the proximal body portion, a proximal end of the first transition wall or the second transition wall on the proximal side substantially abuts against an inner wall of a firing channel of the proximal connecting member so as to limit articulation of the firing member, and a distal end of the first transition wall or the second transition wall on the distal side substantially abuts against an inner wall of a firing channel of the distal connecting member so as to limit articulation of the firing member.

24. The end effector assembly according to any one of claims 19 to 23, **characterized in that** when the distal execution portion extend along the longitudinal axis, distal edges of the first support wall and the second support wall and an inner wall of a firing channel of the distal execution portion are substantially aligned in a straight line; and proximal ends of the first support wall and the second support wall and an inner wall of a channel supporting relative sliding of the firing member in the proximal body portion are substantially aligned in a straight line.

25. The end effector according to any one of claims 1 to 18, **characterized in that** when the end effector assembly is in a ready-to-load position, the distal execution portion forms, with respect to the longitudinal axis, an included angle that is not 0°.

26. The end effector according to claim 25, **characterized in that** the proximal body portion further comprises a locking member rotatably sleeved on a proximal side thereof, wherein the locking member comprises a first locking portion, and when the end effector assembly is in the ready-to-load position, the first locking portion is engaged with the articulating member, so as to lock the articulating member.

27. The end effector according to claim 26, **characterized in that** the locking member comprises a second locking portion, and when the end effector assembly is in the ready-to-load position, the second locking portion engages with the firing member, so as to lock the firing member.

28. The end effector according to claim 27, **characterized in that** the first locking portion of the locking member is engaged in a locked position with the articulating member, and the second locking portion of the locking member is engaged in a locked position with the firing member, respectively, by means of rotational insertion.

29. The end effector assembly according to claim 27 or 28, **characterized in that** the locking member is formed as a semi-annular sleeve with a notch, the first locking portion is a first protrusion extending circumferentially along a notched side wall of the locking member, and the second locking portion is a second protrusion extending inwardly along an inner wall of the locking member.

30. The end effector assembly according to any one of claims 27 to 29, **characterized in that** the proximal body portion comprises a support main body, wherein the locking member is sleeved on a proximal end of the support main body, and the first locking portion and the second locking portion are each located at a distal end of the locking member.

31. The end effector assembly according to any one of claims 26 to 30, **characterized in that** the locking member further comprises an unlocking driving portion, and when the end effector assembly is in a loading position, the unlocking driving portion is subjected to a circumferential force to drive the locking member to rotate and disengage from the articulating member and the firing member.

32. A surgical instrument, comprising a handle assembly, an elongated body assembly, and an end effector assembly selectively engaged with the elongated body assembly, which are sequentially connected from a proximal end to a distal end, **characterized in that** the end effector assembly is implemented as the end effector assembly according to any one of claims 1 to 31.
